# DEMANDE DE BREVET EUROPEEN

(11) **EP 1 666 085 A1**
(43) Date de publication de la demande: **07.06.2006**
(21) Numéro de dépôt: 04028413.5
(22) Date de dépôt: 01.12.2004
(51) Int. Cl.: A61M 37/00, A61M 5/32

(54) **Dispositif d'injection d'un implant solide ou semi-solide**

(71) Demandeur: Societe de Conseils de Recherches et d'Applications Scientifiques (S.C.R.A.S) SAS, 75016 Paris (FR)
(72) Inventeur: Cherif-Cheikh, Roland, 08860 Castelldefels, Barcelona (ES); Aubert, Christophe, 1588 Cudrefin (CH); Rimlinger, Thierry, 38080 L'Isle d'Abeau (FR); Bonacci, Fabrice, 69800 Saint Priest (FR); Barneaud, Serge, 06260 Puget Theniers (FR)
(74) Mandataire: Thérond, Gérard Raymond

(57) **Abrégé**

Dispositif d'injection d'un implant (6) dans des tissus (50), caractérisé en ce qu'il comprend un corps principal creux (2; 2') auquel est fixée une aiguille creuse (4; 4') dans laquelle est introduit l'implant (6), un corps secondaire (26; 26') disposé coaxialement à l'intérieur du corps principal (2; 2') et entourant l'aiguille (4; 4'), et une tige de piston (36; 36') susceptible de coulisser coaxialement à l'intérieur de ladite aiguille creuse (4; 4'), le dispositif d'injection (1; 1') étant agencé de façon que, lorsqu'il est pressé contre les tissus (50), ledit corps principal (2; 2') coulisse le long du corps secondaire (26; 26') depuis une position proximale vers une position distale pour permettre à l'aiguille (4; 4') de pénétrer dans lesdits tissus (50), le déplacement du corps principal (1; 1') s'accompagnant d'un déplacement concomitant de la tige de piston (36; 36'), et de façon que ladite tige de piston (36; 36') demeure fixe et maintienne l'implant (6) à une profondeur requise dans les tissus (50) jusqu'au retrait de l'aiguille (4; 4') hors desdits tissus (50) lors du retour du corps principal (2; 2') de sa position distale à sa position proximale.

## Description

La présente invention concerne un dispositif d'injection et, en particulier, un dispositif pour l'injection intramusculaire ou sous-cutanée d'un principe actif pharmaceutique à l'état solide ou semi-solide habituellement appelé implant. Plus généralement, l'invention s'applique à l'injection d'un corps solide à destination humaine ou encore animale comme par exemple des puces électroniques utilisées pour l'identification d'un être vivant.

Un dispositif d'injection du genre susmentionné est déjà connu du brevet européen EP 0 783 342 au nom de la société DELAB. Un tel dispositif d'injection comprend un corps principal en deux parties auquel est fixée une aiguille creuse par l'intermédiaire d'une pièce de maintien. Une tige est susceptible de coulisser coaxialement à l'intérieur de l'aiguille et vient en butée contre un implant introduit dans ladite aiguille. Un corps secondaire est disposé coaxialement à l'intérieur du corps principal et entoure l'aiguille, de sorte que cette dernière ne fasse pas saillie avant injection. Le corps secondaire comprend une ou plusieurs fentes en différents endroits de sa longueur qui permettent la réunion des deux parties du corps secondaire par le biais d'éléments de connexion s'étendant radialement. La tige présente un renflement qui sert de moyen d'arrêt du déplacement de ladite tige. La tige est reliée à un piston qui, à sa base, comprend un trou longitudinal. Un élément de guidage est fixé sur le piston et est disposé à l'intérieur du corps principal de manière à guider ledit piston et la tige.

Un inconvénient du dispositif d'injection décrit ci-dessus est qu'il comprend un nombre important de pièces. Il est donc coûteux à fabriquer et difficile à assembler. Par ailleurs, les déplacements relatifs des éléments qui constituent ce dispositif d'injection les uns par rapport aux autres sont complexes, ce qui augmente les risques de dysfonctionnement. Un autre inconvénient peut être vu dans le fait que la configuration du dispositif d'injection avant et après injection n'est pas la même, ce qui rend un tel dispositif particulièrement encombrant et donc difficile à stocker.

La présente invention a pour but de remédier aux problèmes susmentionnés ainsi qu'à d'autres encore en procurant un dispositif d'injection qui comprend notamment un nombre de pièces limité et qui est donc moins coûteux à fabriquer et plus facile à assembler.

A cet effet la présente invention concerne un dispositif d'injection d'un implant dans des tissus vivants, caractérisé en ce qu'il comprend un corps principal creux auquel est fixée une aiguille creuse dans laquelle est introduit l'implant, un corps secondaire disposé coaxialement à l'intérieur du corps principal et entourant l'aiguille, et une tige de piston susceptible de coulisser coaxialement à l'intérieur de ladite aiguille creuse, le dispositif d'injection étant agencé de façon que, lorsqu'il est pressé contre les tissus, ledit corps principal coulisse le long du corps secondaire depuis une position proximale vers une position distale pour permettre à l'aiguille de pénétrer dans lesdits tissus, la tige de piston se déplaçant de façon concomitante et de façon que ladite tige de piston demeure fixe et maintienne l'implant à une profondeur requise dans les tissus jusqu'au retrait de l'aiguille hors desdits tissus lors du retour du corps principal de sa position distale à sa position proximale.

Grâce à ces caractéristiques, la présente invention procure un dispositif d'injection d'un implant comprenant un nombre limité de pièces, donc moins coûteux à fabriquer et plus simple à assembler. Par ailleurs, la configuration du dispositif d'injection selon l'invention est la même avant et après utilisation, le corps principal s'éloignant de sa position proximale pour aller vers une position distale, puis revenant à sa position proximale initiale, de sorte que l'encombrement du dispositif d'injection est limité, ce qui facilite le conditionnement et le stockage de tels dispositifs.

Selon une caractéristique complémentaire de l'invention, la tige de piston est entraînée par le corps principal lorsque celui-ci se déplace de sa position proximale vers sa position distale, ladite tige de piston étant découplée dudit corps principal lorsque ce dernier revient de sa position distale à sa position proximale.

Grâce à cette autre caractéristique, les mouvements relatifs des pièces qui composent le dispositif d'injection les unes par rapport aux autres sont simplifiés, ce qui permet d'éviter les risques de dysfonctionnement du dispositif.

Selon encore une autre caractéristique de l'invention, le dispositif d'injection comprend un mécanisme qui prévient son verrouillage avant utilisation et qui autorise ce verrouillage après utilisation.

Ainsi, avant utilisation, il n'est pas possible de verrouiller le dispositif d'injection par inadvertance, tandis qu'après utilisation, le corps secondaire vient se verrouiller de manière irréversible sur le corps principal, ce qui rend impossible toute utilisation ultérieure du dispositif d'injection selon l'invention. En outre, tout risque de blessure accidentelle pouvant occasionner une contamination du praticien est évité.

D'autres caractéristiques et avantages de la présente invention ressortiront plus clairement de la description détaillée qui suit d'un mode de réalisation du dispositif d'injection selon l'invention, cet exemple étant donné à titre purement illustratif et non limitatif seulement, en liaison avec le dessin annexé sur lequel :
- la figure 1 est une vue générale en perspective du dispositif d'injection selon l'invention;
- la figure 2 est une vue en perspective et en éclaté du dispositif de la figure 1;
- la figure 3A est une vue en coupe longitudinale en position de repos du dispositif d'injection selon l'invention;
- la figure 3B est une vue analogue à celle de la figure 3A, le plan de coupe ayant été tourné de 90°;
- les figures 4A, 4B, 4C et 4D illustrent les différentes phases de fonctionnement du dispositif d'injection selon l'invention;
- les figures 5A, 5B, 5C et 5D sont des représentations schématiques qui illustrent le mode d'emploi du dispositif d'injection selon l'invention;
- la figure 6 est une vue de détail à plus grande échelle montrant le couplage de la tige de piston avec le corps secondaire lors de la descente du corps principal vers sa position distale;
- la figure 7 est une vue de détail montrant le découplage de la tige de piston et du corps secondaire lors du retour du corps principal vers sa position proximale;
- les figures 8A, 8B sont des vues en coupe longitudinale de l'extrémité proximale du dispositif d'injection qui illustrent le blocage temporaire unidirectionnel du corps secondaire relativement au corps principal avant utilisation;
- les figures 9A, 9B sont des vues analogues à celles des figures 8A, 8B qui illustrent le verrouillage irréversible du corps secondaire sur le corps principal après injection;
- les figures 10A, 10B et 10C illustrent différentes variantes de réalisation de la tige de piston;
- la figure 11A est une vue en coupe longitudinale de l'extrémité proximale du dispositif d'injection selon un second mode de réalisation autorisant le chargement de l'implant par l'arrière du dispositif d'injection;
- la figure 11B est une vue d'une variante de réalisation de l'extrémité proximale du dispositif d'injection illustrée à la figure 11A;
- la figure 12A est une vue en perspective de l'extrémité proximale du dispositif d'injection selon le second mode de réalisation montrant le chargement de l'implant et de la tige de piston;
- la figure 12B est une vue en coupe longitudinale du dispositif d'injection selon le second mode de réalisation;
- la figure 13A illustre un mode particulier de réalisation de la butée supérieure prévue à l'extrémité proximale du corps principal pour autoriser l'introduction de la tige de piston et en interdire son extraction lors du chargement par l'arrière du dispositif d'injection;
- les figures 13B, 13C et 13D illustrent différentes variantes de réalisation de la tige de piston compatibles avec un chargement par l'arrière du dispositif d'injection;
- la figure 14 est une vue en perspective de la pièce de maintien de l'aiguille creuse;
- la figure 15 est une vue de dessus d'un premier mode de réalisation des moyens de verrouillage irréversible du corps secondaire sur le corps principal;
- la figure 16 est une vue en perspective d'un premier mode de réalisation des moyens prévus sur le corps secondaire pour le blocage de la tête de la tige de piston;
- la figure 17C est une vue à plus grande échelle de la zone entourée d'un cercle sur la figure 17B qui montre la position d'une languette par rapport à un chemin de came lorsque le dispositif d'injection est dans sa position initiale avant utilisation comme représenté en coupe à la figure 17A;
- les figures 18A à 18C sont des vues analogues à celles des figures 17A à 17C, le corps principal ayant été coulissé le long du corps secondaire pour permettre à l'aiguille de commencer à pénétrer dans la peau, la languette se déplaçant le long du chemin de came;
- les figures 19A à 19C sont des vues analogues à celles des figures 17A à 17C, le corps principal ayant été amené à sa position distale extrême dans laquelle l'aiguille est complètement enfoncée dans la peau, la languette ayant atteint son point de rebroussement;
- les figures 20A à 20C sont des vues analogues à celles des figures 17A à 17C, le corps principal étant remonté partiellement le long du corps secondaire pour permettre à l'aiguille de sortir de la peau, la languette se déplaçant le long du côté opposé du chemin de came;
- les figures 21A à 21C sont des vues analogues à celles des figures 17A à 17C, le dispositif d'injection étant dans sa position verrouillée après utilisation, la languette étant tombée dans un logement prévu à l'extrémité du chemin de came pour empêcher la désolidarisation du corps secondaire et du corps principal;
- les figures 22A à 22D illustrent les différentes phases de fonctionnement d'un deuxième mode de réalisation du dispositif d'injection selon l'invention;
- les figures 23A et 23B sont des vues en coupe longitudinale, respectivement avant et après utilisation, d'une variante simplifiée de réalisation du dispositif d'injection selon l'invention, et
- les figures 24A à 24D sont des représentations schématiques qui illustrent le principe de fonctionnement d'une variante de réalisation des moyens de verrouillage du corps secondaire sur le corps principal.

La présente invention procède de l'idée générale inventive qui consiste à procurer un dispositif d'injection d'un corps solide ou semi-solide à visée thérapeutique ou non qui comprenne notamment un nombre peu élevé de pièces, rendant son montage plus facile et son fonctionnement plus sûr. La présente invention concerne également un tel dispositif d'injection qui puisse être verrouillé de manière irréversible après injection pour rendre impossible toute utilisation ultérieure d'un tel dispositif et éviter tout risque de blessure accidentelle pouvant occasionner une contamination.

Dans tout ce qui suit, on entendra par extrémité "proximale" l'extrémité par laquelle l'utilisateur se saisit du dispositif d'injection, et par extrémité "distale" l'extrémité du dispositif d'injection située du côté du biseau de l'aiguille.

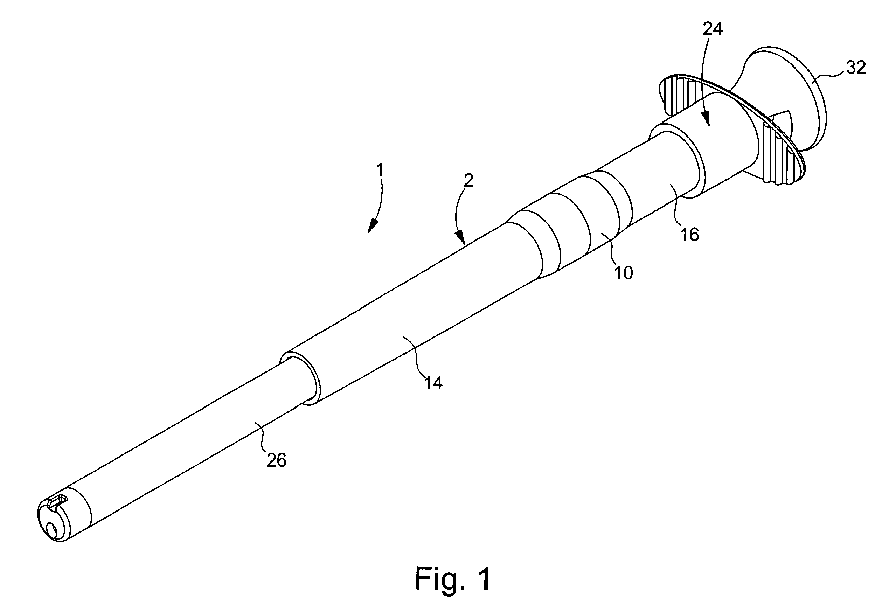

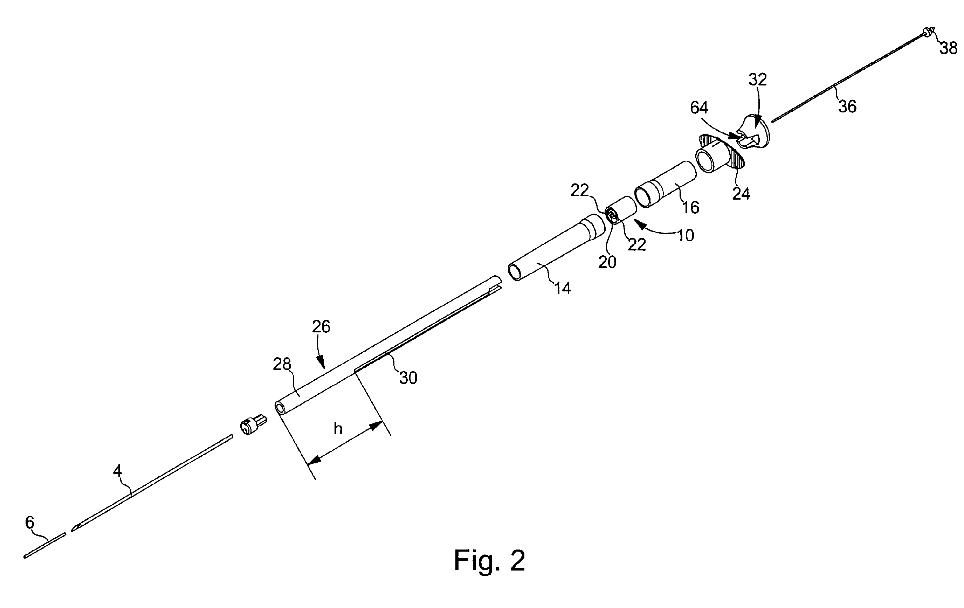

Un premier mode d'exécution du dispositif d'injection selon l'invention va être décrit en relation avec les figures 1 à 3B annexées à la présente demande de brevet.

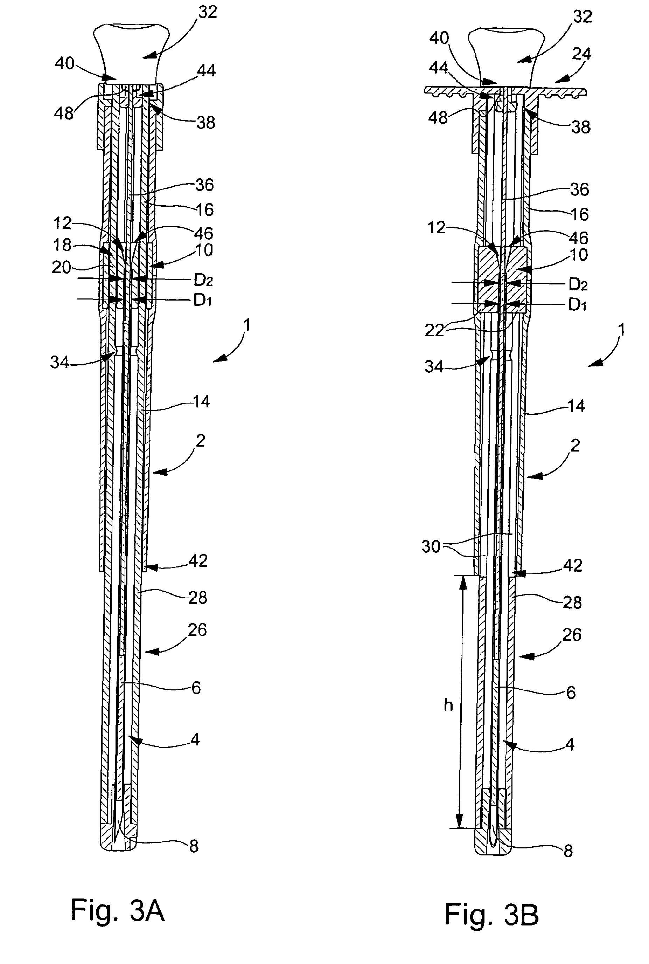

Désigné dans son ensemble par la référence numérique générale 1, le dispositif d'injection représenté en coupe aux figures 3A et 3B comprend un corps principal creux 2 auquel est fixée de manière permanente une aiguille creuse 4 dans laquelle est introduit un implant 6 destiné à être injecté. Les implants injectables avec un dispositif selon l'invention comprennent aussi bien des compositions pharmaceutiques solides ou semi-solides que d'autres implants à but non thérapeutique comme, par exemple, des puces électroniques utilisées pour l'identification d'êtres vivants.

De préférence, l'aiguille creuse 4 présente à son extrémité distale un biseau 8 dont la géométrie est adaptée à l'application prévue pour le dispositif d'injection 1 et, en particulier, au mode d'administration. A son extrémité proximale, l'aiguille creuse 4 est montée fixe dans une pièce de maintien 10. Une ouverture traversante 12 est pratiquée dans la pièce de maintien 10 pour le montage de l'aiguille 4. Cette ouverture traversante 12 présente une première partie de diamètre D₁ égal ou légèrement supérieur au diamètre extérieur de l'aiguille 4 pour permettre d'engager l'extrémité proximale de cette dernière dans la pièce de maintien 10. La première partie de diamètre D₁ de l'ouverture traversante 12 est suivie d'une seconde partie de diamètre D₂ inférieur au diamètre extérieur de l'aiguille 4 pour permettre le maintien de ladite aiguille 4 dans la pièce de maintien 10 par frottement. Cette immobilisation de l'aiguille 4 pourra être renforcée par collage ou autre. Enfin, la seconde partie de diamètre D₂ de l'ouverture traversante 12 est suivie d'une troisième partie en forme de cône qui va en s'évasant dans la direction de l'extrémité proximale du dispositif d'injection 1 selon l'invention et qui permet l'insertion et le bon guidage de la tige de piston 36.

Selon une première variante de réalisation (non représentée) de la pièce de maintien 10, celle-ci est faite d'une pièce avec le corps principal creux 2. Selon une seconde variante de réalisation représentée au dessin, la pièce de maintien 10 sert de pièce de jonction à des parties tubulaires inférieure 14 et supérieure 16 qui constituent le corps principal creux 2 et dans lesquelles ladite pièce de maintien 10 est enchâssée. Dans l'exemple représenté au dessin, la pièce de maintien 10 est une pièce de révolution. Il va de soi que si le corps principal 2 devait par exemple présenter un méplat pour faciliter le maniement du dispositif d'injection 1 et donner au praticien une indication relative au bon positionnement de ce dispositif 1 préalablement à l'injection, la pièce de maintien 10 pourrait s'écarter de sa forme cylindrique.

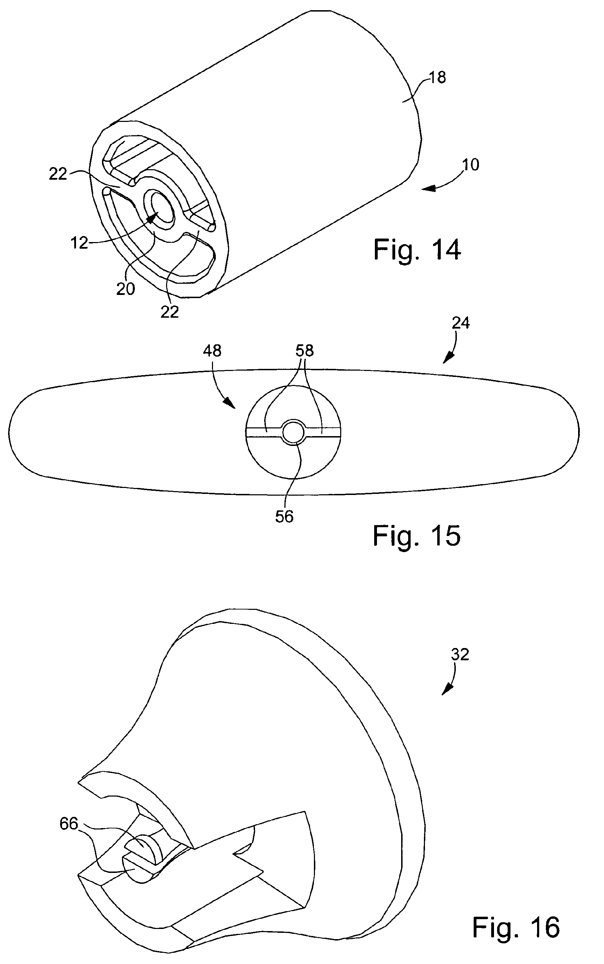

La pièce de maintien 10 comprend une paroi latérale extérieure 18 qui délimite un volume intérieur dans lequel l'ouverture traversante 12 s'étend coaxialement. L'ouverture traversante 12 est matérialisée par une portion de tube 20 qui est reliée à la paroi extérieure 18 par au moins une et, préférentiellement, deux nervures radiales 22 diamétralement opposées (voir également figure 14).

De façon avantageuse, l'extrémité proximale du corps principal 2 est munie d'une pièce appuie-doigts 24 venant de matière avec ledit corps principal 2 ou se présentant sous forme d'une pièce rapportée.

Le corps principal creux 2 est apte à coulisser le long d'un corps secondaire 26, également creux, disposé coaxialement à l'intérieur dudit corps principal 2 et entourant l'aiguille 4. Ce corps secondaire 26 se présente sous la forme générale d'un tube 28 pourvu de deux fentes rectilignes 30 diamétralement opposées et qui s'étendent depuis l'extrémité proximale du tube 28 jusqu'à une hauteur h au-dessus de l'extrémité distale dudit tube 28 qui détermine la profondeur de pénétration de l'aiguille 4. L'extrémité proximale du tube 28 sera avantageusement équipée de moyens facilitant la préhension du dispositif d'injection 1 tels qu'un bouton 32. Ce bouton 32 pourra être fait d'une pièce avec le corps secondaire 26 ou bien être réalisé par une pièce rapportée. Enfin, le corps secondaire 26 présente sur la face intérieure du tube 28 des moyens de blocage 34, par exemple sous la forme de deux bourrelets dont le rôle sera décrit ci-après.

Le dispositif d'injection 1 selon l'invention comprend enfin une tige de piston 36 susceptible de coulisser à l'intérieur de l'aiguille creuse 4 et qui sert à maintenir l'implant 6 à la bonne profondeur dans les tissus. Cette tige de piston 36 comprend une tête 38 qui coopère avec les moyens de blocage 34 prévus sur le corps secondaire 26 pour permettre son découplage du corps principal 2 comme il sera décrit en détail ci-après.

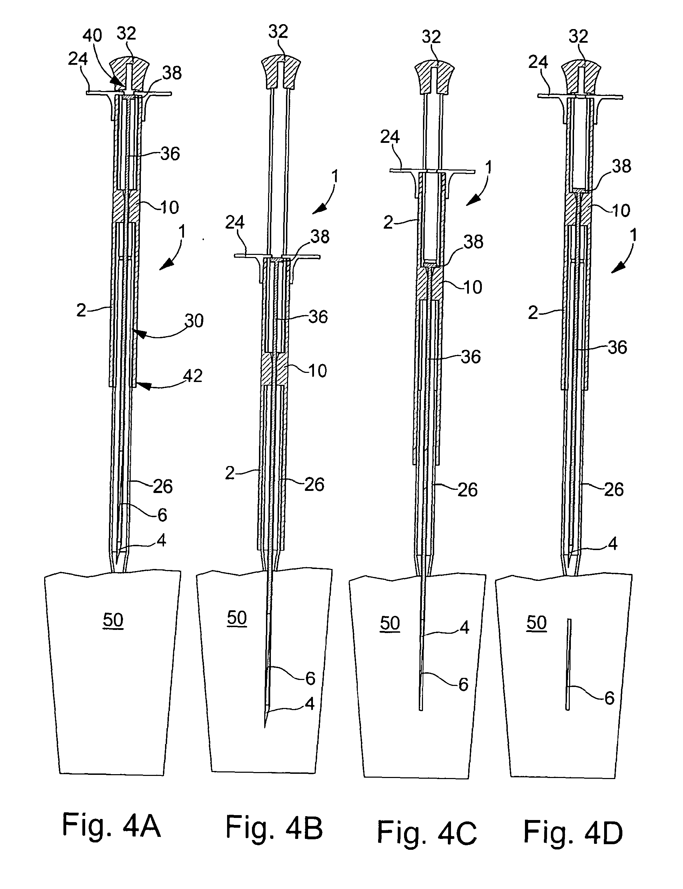

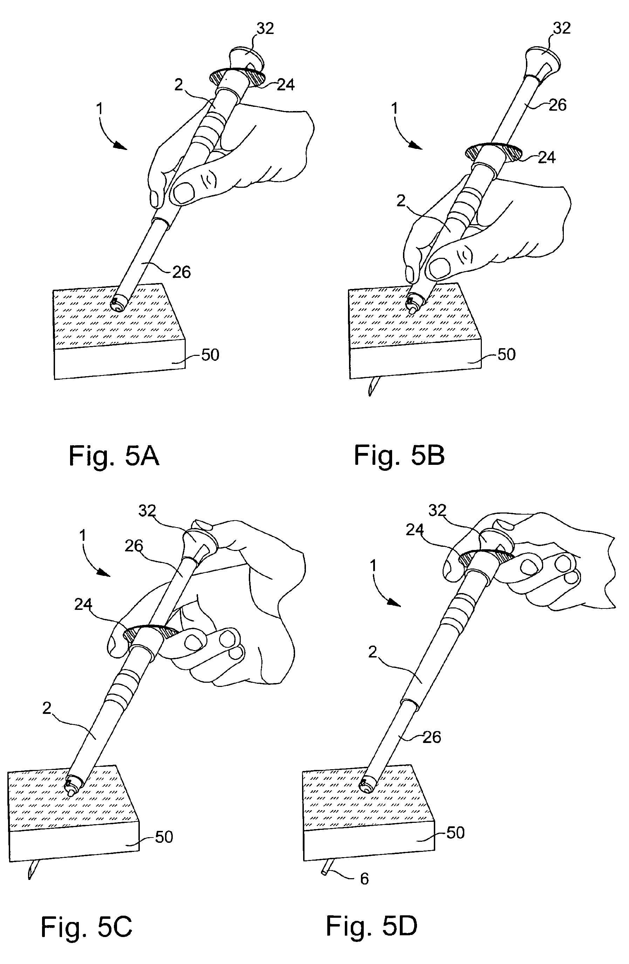

On s'intéresse maintenant au principe de fonctionnement du dispositif d'injection 1 en se référant plus particulièrement aux figures 4A à 4D et 5A à 5D.

On notera tout d'abord que le corps principal 2 ne peut s'ébattre qu'entre une position proximale et une position distale contrôlées respectivement par une butée supérieure 40 et une butée inférieure 42 ménagées sur le corps secondaire 26. Dans l'exemple représenté au dessin (voir notamment la figure 3A), la butée supérieure est formée par le bouton 32 contre lequel le corps principal 2 vient buter par son appuie-doigts 24. Quant à la butée inférieure 42, elle est constituée par les fonds des fentes 30 contre lesquels le corps principal 2 vient buter par ses nervures 22 lorsqu'il coulisse axialement le long du corps secondaire 26.

De même, la tige de piston 36 ne peut s'ébattre qu'entre une position proximale et une position distale contrôlées respectivement par une butée supérieure 44 et une butée inférieure 46 ménagées sur le corps principal 2. Dans l'exemple représenté au dessin, la butée supérieure 44 est constituée par des moyens 48 qui permettent le blocage irréversible du corps secondaire 26 sur le corps principal 2 après injection. Ces moyens 48 qui seront décrits en détail ultérieurement peuvent être faits d'une pièce avec l'appuie-doigts 24 dans le cas où le dispositif d'injection 1 comprend un tel élément. Quant à la butée inférieure 46, elle est constituée par la face supérieure de la pièce de maintien 10 de l'aiguille 4.

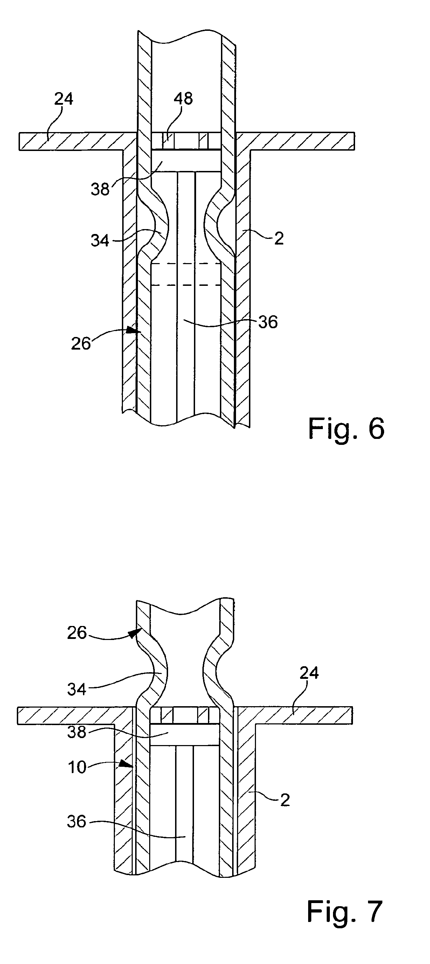

Aux figures 4A, 5A, le dispositif d'injection 1 selon l'invention est représenté tel qu'il est livré au praticien, le corps principal 2 occupant sa position proximale au voisinage du bouton 32 et le corps secondaire 26 entourant l'aiguille 4 jusqu'à son biseau 8, de sorte que tout risque d'éraflure est évité. De même, l'aiguille 4 est cachée à la vue du patient avant injection. En tenant d'une main le dispositif d'injection 1 par son corps principal 2, le praticien vient appuyer l'extrémité distale du corps secondaire 26 contre la peau 50 du patient. Lorsque le dispositif d'injection 1 est convenablement disposé, le praticien exerce une poussée sur le corps principal 2. Sous l'effet de cette poussée, le corps principal 2 se met à coulisser axialement le long du corps secondaire 26, permettant à l'aiguille 4 qui est solidaire dudit corps principal 2 de pénétrer dans la peau 50. Dans le même temps, le corps principal 2 entraîne la tige de piston 36 par le biais de la face inférieure de ses moyens de blocage 48, de sorte que la position de ladite tige 36 relativement à l'aiguille 4 et l'implant 6 reste inchangée. Peu de temps avant que le corps principal 2 n'atteigne sa position de butée distale, la tête 38 de la tige de piston 36 arrive à la hauteur des moyens de blocage 34 que porte le corps secondaire 26 sur sa surface inférieure et les dépasse (voir figure 6). Ce mouvement de la tête 38 de la tige de piston 36 relativement aux moyens de blocage 34 est rendu possible grâce au fait que ladite tige de piston 36 est immobilisée contre la butée supérieure 44 ménagée sur le corps principal 2 et que les moyens de blocage 34 sont aptes à se déformer élastiquement pour laisser le passage à ladite tête 38. Après que les moyens de blocage 34 soient passés derrière la tête 38 de la tige de piston 36, le corps principal 2 atteint sa position de butée distale définie par les nervures radiales 22 qui arrivent en fond de fentes 30. A ce moment, l'aiguille creuse 4 a pénétré au maximum dans la peau 50 du patient (figures 4B, 5B).

C'est à ce stade que survient l'opération de rétro-injection proprement dite de l'implant 6 (figures 4C, 5C), c'est-à-dire le dépôt à une profondeur déterminée et indépendante des circonstances dudit implant 6. En effet, le praticien, dans un geste qui n'est pas sans rappeler celui d'une injection classique va, de son pouce, maintenir le dispositif d'injection 1 contre la peau 50 du patient au moyen du bouton 32 tandis que, de son index et de son majeur, il va commander le retour du corps principal 2 vers sa position de butée proximale en se servant de l'appuie-doigts 24. Au cours de ce mouvement, le corps principal 2 coulisse le long du corps secondaire 26, ce qui provoque le retrait progressif de l'aiguille 4 hors de la peau 50 du patient. La tige de piston 36 n'accompagne toutefois pas ce mouvement de recul du corps principal 2. En effet, retenue par les moyens de blocage 34 prévus sur le corps secondaire 26, elle est découplée dudit corps principal 2 et reste immobile, pénétrant ainsi progressivement dans l'aiguille creuse 4 au fur et à mesure que cette dernière sort de la peau 50. L'implant 6 émerge ainsi de l'aiguille 4, maintenu en position à la bonne profondeur dans la peau 50 par l'extrémité distale de la tige de piston 36 qui est en appui contre ledit implant 6. On notera que la longueur de la tige de piston 36 est telle que, lorsque l'aiguille 4 s'est déplacée relativement à cette tige de piston 36, celle-ci ne dépasse pas de la pointe de ladite aiguille 4, mais est au moins affleurant avec le talon du biseau 8 ou tout au moins suffisamment proche dudit biseau 8 pour que l'implant 6, déjà largement engagé dans les tissus 50, ne nécessite plus cet appui.

L'opération d'injection s'achève lorsque le corps principal 2 est de retour à sa position de butée proximale (figures 4D, 5D). Au cours du mouvement de remontée du corps principal 2 relativement au corps secondaire 26, la face supérieure de la pièce de maintien 10 de l'aiguille 4 vient à la rencontre de la tête 38 de la tige de piston 36 qu'elle bloque. Comme lors du mouvement de descente du corps principal 2, les moyens de blocage 34 que porte le corps secondaire 26 vont dépasser la tête 38 de la tige de piston 36 en se déformant élastiquement pour autoriser la poursuite du mouvement d'ascension dudit corps principal 2 (voir figure 7). Finalement, le corps principal 2 vient se verrouiller de manière irréversible sur le corps secondaire 26 par le biais de ses moyens de blocage 48. On remarquera que l'encombrement du dispositif d'injection 1 est le même avant et après utilisation, ce qui en facilite notamment la manipulation par le praticien et le stockage.

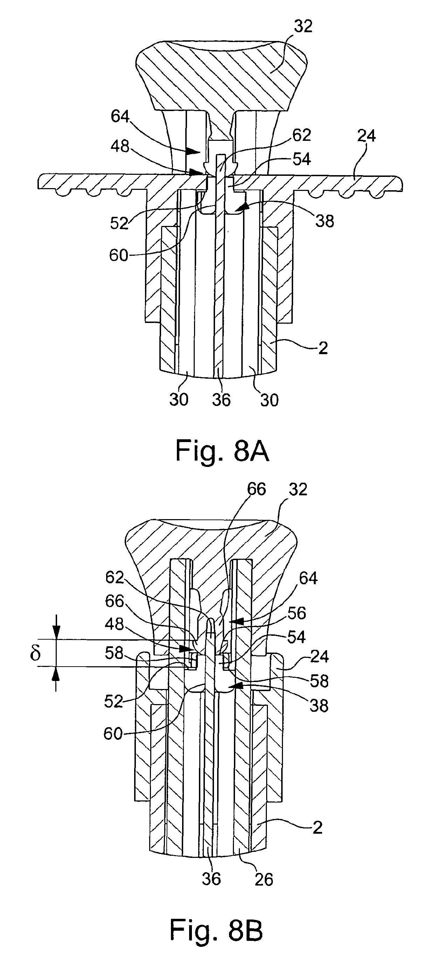

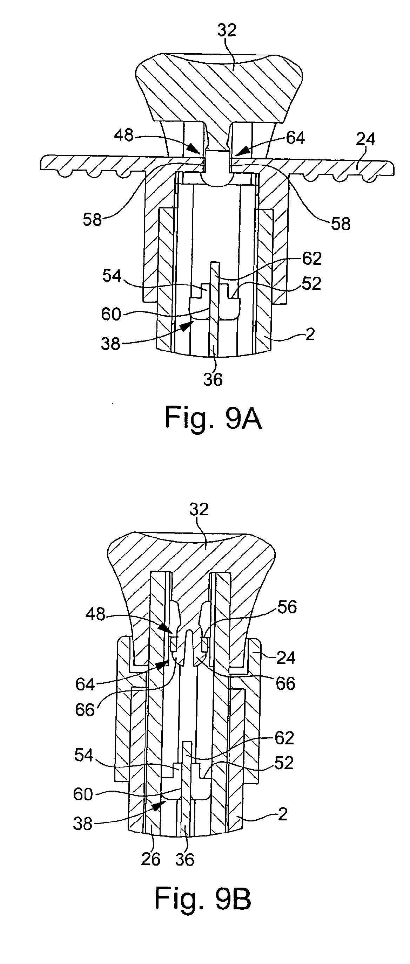

On s'intéresse maintenant en liaison avec les figures 8A, 8B et 9A, 9B au blocage et au verrouillage du dispositif d'injection 1 selon l'invention respectivement avant et après utilisation.

Comme on peut le voir à l'examen des figures 8A et 8B, la tête 38 de la tige de piston 36 est une pièce de révolution qui présente à son extrémité proximale un épaulement annulaire 52 définissant un téton 54 par l'intermédiaire duquel la tête 38 coopère avec les moyens de blocage 48 ménagés sur le corps principal 2. Ces moyens de blocage 48 comprennent un anneau circulaire 56 relié à l'appuie-doigts 24 par le biais d'au moins un et, préférentiellement, de deux bras radiaux 58 diamétralement opposés (voir également figures 15 et 16). Dans le cas où le dispositif d'injection ne comprendrait pas de pièce appuie-doigts 24, les moyens de blocage 48 pourraient être réalisés d'une pièce avec le corps principal 2. On notera que la tête 38 présente un trou traversant 60 dans lequel la tige de piston 36 est enfilée de manière que son extrémité proximale 62 fasse saillie. Selon l'invention, la tête 38 de la tige de piston 36 est engagée par le téton 54 dans l'ouverture circulaire définie par l'anneau 56 et vient buter contre les bras 58 par son épaulement 52. Dans le même temps, l'extrémité proximale 62 de la tige de piston 36 coopère avec des moyens de verrouillage 64 qui, selon un mode de réalisation préféré mais non limitatif de l'invention, sont faits d'une pièce avec le bouton 32. Ces moyens de verrouillage 64 comprennent, dans l'exemple représenté au dessin, une paire de clips 66 entre lesquels est engagée l'extrémité proximale 62 de la tige de piston 36. Ce faisant, l'extrémité 62 de la tige 36 écarte légèrement les clips 66 de leur position de repos en les déformant élastiquement en extension, ce qui interdit à ces clips 66 de pénétrer dans l'ouverture circulaire définie par l'anneau 56. Ainsi, avant utilisation, le dispositif d'injection 1 selon l'invention est bloqué dans le sens du verrouillage, le corps secondaire 26 ne pouvant s'enfoncer dans le corps principal 2.

Au début de l'opération d'injection, on fait coulisser le corps principal 2 le long du corps secondaire 26 pour enfoncer l'aiguille 4 dans la peau 50. Le corps principal 2 entraîne ainsi avec lui la tige de piston 36, de sorte que l'extrémité 62 de cette tige 36 se dégage des clips 66 qui reviennent à leur position de repos. A la fin de l'opération d'injection, le corps principal 2 est ramené vers sa position proximale en coulissant le long du corps secondaire 26, la tige de piston 36 étant découplée dudit corps principal 2 comme décrit ci-dessus. Les clips 66 n'étant plus entravés par l'extrémité 62 de la tige de piston 36, ils peuvent franchir la distance δ (voir figure 8A) les séparant de la base de l'ouverture délimitée par l'anneau 56 et pénétrer dans cette dernière en se déformant élastiquement en compression, puis recouvrent leur position de repos lorsqu'ils émergent de ladite ouverture (voir figures 9A et 9B). Ainsi, après utilisation, le corps secondaire 26 est verrouillé de manière irréversible sur le corps principal 2, ce qui empêche toute utilisation ultérieure du dispositif d'injection 1 selon l'invention. De même, tout risque de piqûre pouvant occasionner une contamination est écarté.

Selon un mode de réalisation préféré, la pièce appuie-doigts 24 sera opaque et la tête 38 de la tige de piston 36 sera colorée, par exemple de couleur rouge. Avant utilisation du dispositif d'injection 1, la tête 38 ne sera donc pas visible, ce qui fournit au praticien une indication relative à l'état non utilisé dudit dispositif 1. Parallèlement, le corps principal 2 peut être transparent ou opaque et comprendre une fenêtre à travers laquelle la tête 38 de la tige de piston 36 sera visible après utilisation, ce qui indiquera au praticien que le dispositif d'injection 1 est déchargé.

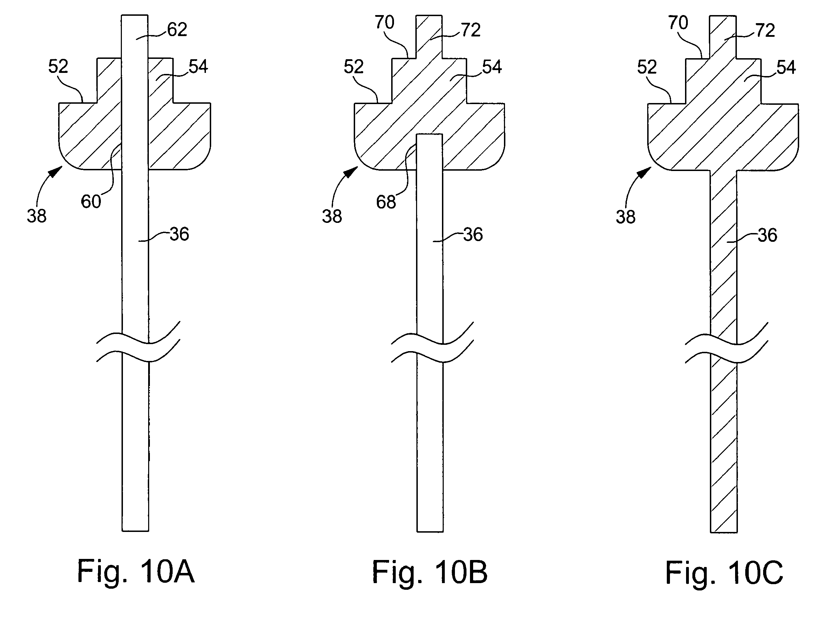

Différentes variantes de réalisation de la tige de piston 36 sont illustrées aux figures 10A à 10C. La variante de réalisation représentée à la figure 10A vient d'être décrite ci-dessus. On rappellera simplement que la tête 38 de la tige 36 présente un épaulement annulaire définissant un téton 54 par lequel elle coopère avec les moyens de blocage 48 prévus sur le corps principal 2, ainsi qu'un trou traversant 60 dans lequel ladite tige 36 est enfilée de manière que son extrémité proximale 62 fasse saillie.

Selon une seconde variante de réalisation illustrée à la figure 10B, la tête 38 de la tige de piston 36 comprend un trou borgne 68 dans lequel est engagée ladite tige 36. La tête 38 comprend par ailleurs un épaulement supplémentaire 70 qui définit une excroissance 72 qui joue, vis-à-vis des moyens de verrouillage 64, le même rôle que l'extrémité proximale 62 de la tige de piston 36.

Enfin, la tige de piston 36 illustrée à la figure 10C est de mêmes formes et dimensions que celle représentée à la figure 10B, à ceci près que la tête 38 et la tige 36 sont faites d'une seule pièce, par exemple par injection d'une matière plastique.

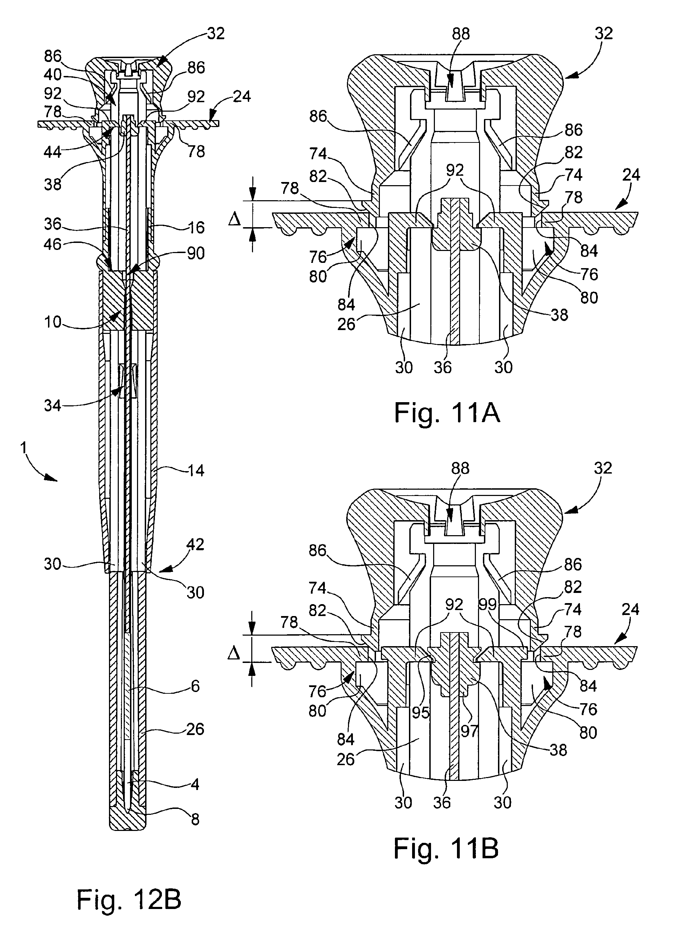

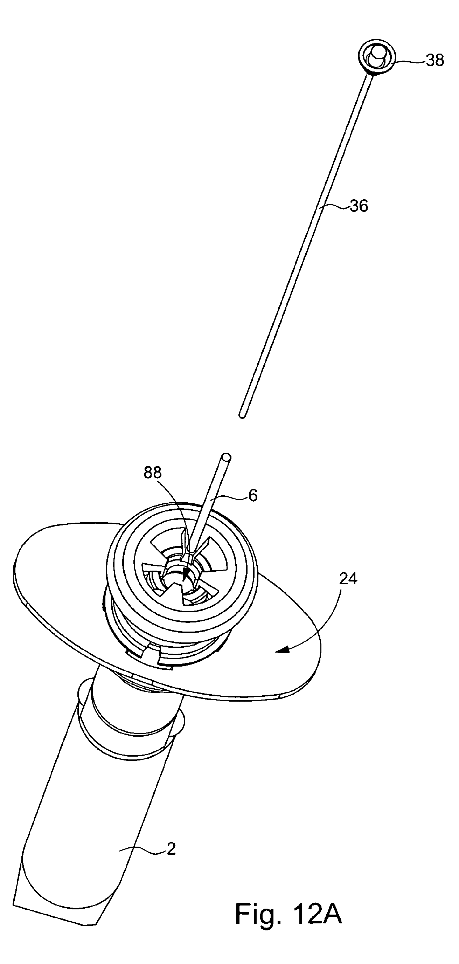

Avantageusement, les dispositifs d'injection d'implants selon l'invention sont commercialisés pré-remplis. En d'autres termes, ils contiennent déjà l'implant à administrer au patient. Dans tout ce qui vient d'être décrit, le chargement de l'implant 6 se fait par l'avant, c'est-à-dire par l'extrémité distale de l'aiguille creuse 4, du côté du biseau 8 de cette dernière, en raison de la présence des clips 66 sur l'axe de symétrie longitudinale du dispositif d'injection 1. Le chargement par l'avant présente, lorsque la pointe de l'aiguille 4 est biseautée, un risque de piqûre pour l'opérateur dans le cas d'un chargement manuel, de contamination de l'aiguille 4, de dommage à l'implant 6 ou encore au biseau 8. C'est pourquoi le chargement par l'arrière (extrémité proximale) sera privilégié lorsqu'il est possible. Un mode de réalisation qui satisfait à ces exigences est illustré aux figures 11A, 11 B et 12A, 12B.

Comme on peut le constater à l'examen de la figure 11A, au moins deux clips 74 présentant une résistance importante à l'enfoncement (par exemple de 40 à 60 N et, de préférence, de l'ordre de 50 N) sont ménagés en périphérie inférieure du bouton 32 ou directement sur le corps secondaire 26 lorsque ce dernier n'est pas muni d'un tel moyen en facilitant la préhension. Ces clips 74 coopèrent avec des éléments de retenue 76 ménagés à l'extrémité proximale du corps principal 2. Ces éléments de retenue 76 présentent une forme générale de L inversé et comprennent chacun un retour 78 qui délimite une cavité 80 ouverte du côté intérieur du corps principal 2. La résistance à l'enfoncement des clips 74 est telle qu'avant utilisation du dispositif d'injection 1, le déplacement sur une distance Δ du corps secondaire 26 relativement au corps principal 2 qui permettrait le verrouillage des clips 74 sur les éléments de retenue 76 est rendu difficile à moins d'exercer une force supérieure à la résistance à l'enfoncement de ces clips 74. Le corps secondaire 26 ne peut donc être verrouillé sur le corps principal 2 par inadvertance. Après avoir réalisé l'injection, le praticien exerce une pression suffisante pour verrouiller lesdits clips 74. Au cours de cette opération, les clips 74 glissent par leurs plans inclinés 82 sur les arêtes 84 en regard des retours 78 en se déformant élastiquement et recouvrent leur forme initiale en pénétrant dans les cavités 80. Il va de soi que la géométrie des moyens de verrouillage du corps secondaire 26 sur le corps principal 2 pourrait être inversée, les cavités 80 étant alors ouvertes du côté extérieur du corps principal 2 et les clips 74 venant enserrer les éléments de retenue 76. Dans ce mode de réalisation, la tige de piston 36 est en appui contre l'implant 6, ce qui l'empêche de tomber. La tige de piston 36 fournit donc une indication sur la présence de l'implant. Si l'implant 6 tombe ou si le dispositif d'injection 1 n'est pas chargé, la tige de piston 36 tombe sous l'effet de la gravitation lorsque le dispositif d'injection 1 est maintenu en position verticale.

Le bouton 32 peut être collé sur le corps secondaire 26. Selon une variante préférée, le bouton 32 est rendu solidaire du corps secondaire 26 de manière irréversible par encliquetage au moyen de cliquets 86.

Grâce à ce mode de réalisation dans lequel les moyens permettant le verrouillage du corps secondaire 26 sur le corps principal 2 sont éloignés du centre du dispositif d'injection 1, le chargement par l'arrière de ce dernier peut être envisagé. A cet effet, le bouton 32 présente une ouverture traversante 88 par laquelle peuvent successivement être engagés l'implant 6 et la tige de piston 36 (figures 12A, 12B). A cet effet, l'ouverture 12 qui traverse la pièce de maintien 10 de l'aiguille 4 présentera à son extrémité proximale une forme en cône 90 s'évasant vers le haut pour faciliter l'introduction dudit implant 6 et de ladite tige de piston 36. Ainsi, le dispositif d'injection 1 selon l'invention peut être livré au fabricant d'implants avec la tige de piston 36 séparée dudit dispositif 1. Le fabricant d'implants n'aura donc plus qu'à charger l'implant 6 par l'extrémité proximale dudit dispositif 1 puis à introduire ladite tige 36.

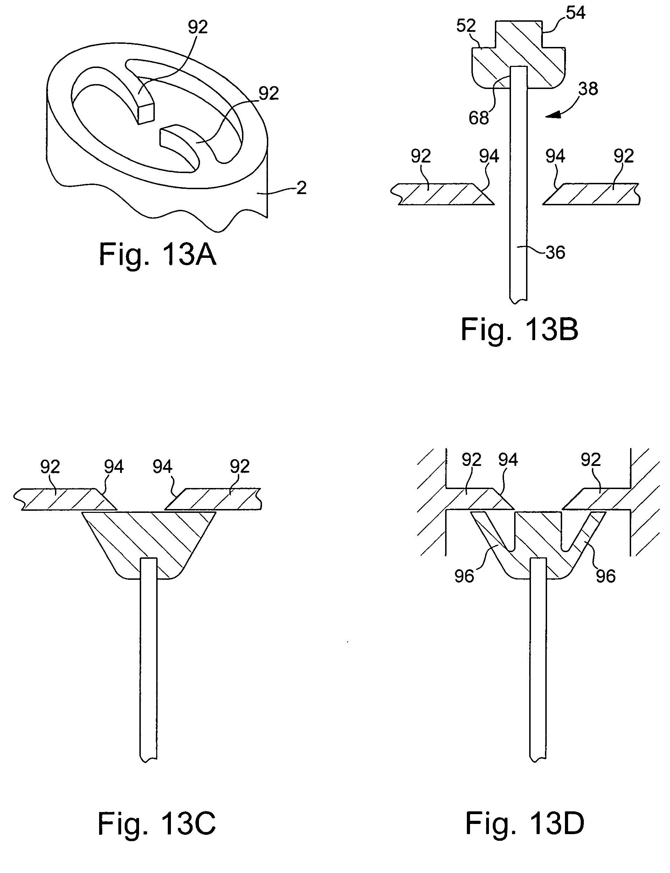

Il est toutefois nécessaire que la tige de piston 36, une fois introduite, ne puisse plus être extraite sans effort du dispositif d'injection 1. Ceci peut être réalisé en donnant à la tête 38 de la tige de piston 36 et à l'extrémité proximale du corps principal 2 des géométries empêchant ce retrait. Selon un premier mode de réalisation (voir figure 13B), la tête 38 de la tige 36 a une forme analogue à celle décrite ci-dessus, exception faite du téton 54 qui est supprimé, car sans objet. La tête 38 de la tige 36 coopère avec deux bras 92 diamétralement opposés prévus à l'extrémité proximale du corps principal 2 et qui se déforment élastiquement vers le bas lors du passage de la tête 38 (voir figure 13A). Lorsque la tête 38 a dépassé les bras 92, ceux-ci recouvrent leur position initiale, empêchant l'extraction de la tige de piston 36. Pour faciliter l'introduction de la tige de piston 36, les bras 92 présentent deux plans inclinés 94 dans le sens de l'avance de ladite tige 36. Selon un deuxième mode de réalisation (voir figure 13C), la tête 38 de la tige de piston 36 aura une forme tronconique et sera réalisée dans une matière flexible, tandis que les bras 92 seront réalisés au moyen d'une matière rigide. Selon un troisième mode de réalisation, la tête 38 de la tige de piston 36 présente deux bras inclinés 96 qui convergent vers l'extrémité distale du dispositif d'injection 1 et qui se déforment élastiquement lors de leur passage à hauteur des bras 92 en regard ménagés sur le corps principal 2 (voir figure 13D).

Comme on peut le voir à l'examen de la figure 11 B, la tête 38 de la tige de piston 36 peut présenter une gorge circulaire périphérique 95 dans laquelle les plans inclinés 94 des bras 92 font saillie. Ainsi, même en l'absence de l'implant 6, la tige de piston 36 ne peut tomber sous l'effet de la gravitation lorsque le dispositif d'injection 1 est maintenu verticalement. De même, selon cette variante de réalisation, la tête 38 de la tige de piston 36 présente à sa base un téton 97 adapté en forme et en dimension pour venir se loger dans l'ouverture en forme de cône 90 que présente l'ouverture 12 pratiquée dans la pièce de maintien 10 à son extrémité proximale. Ainsi, après injection de l'implant 6, la tige de piston 36 est immobilisée sur la pièce de maintien 10. Enfin, le corps principal 2 présente un rebord périphérique extérieur 99 agencé de sorte que l'espace laissé libre entre ce rebord 99 et la pièce appuie-doigts 24 est tel qu'il interdit le passage des clips 74 avant activation du dispositif d'injection 1.

Selon un mode de réalisation préféré, la pièce appuie-doigts 24 sera opaque et comprendra une fenêtre à travers laquelle la tête 38 de la tige de piston 36 apparaîtra, indiquant au praticien que le dispositif d'injection 1 est chargé. En effet, comme la tige de piston 36 est libre dans l'aiguille 4 et uniquement en appui contre l'implant 6, sa tête 38 n'apparaîtra dans la fenêtre de visualisation pratiquée dans le corps secondaire 26 que si ledit implant 6 est présent dans ladite aiguille 4. Après injection, la tête 38 de la tige de piston 36 apparaîtra dans une seconde fenêtre pratiquée dans le corps principal opaque 2 pour indiquer au praticien que le dispositif d'injection 1 a été utilisé et est donc vide. On pourrait également disposer d'une fenêtre s'étendant depuis la position qu'occupe la tête 38 de la tige de piston 36 lorsque le dispositif d'injection 1 est chargé jusqu'à la position qu'occupe cette tête 38 lorsque ledit dispositif d'injection 1 est vide. De la sorte, il serait possible de visualiser la progression de l'opération de rétro-injection.

Selon une variante de réalisation de l'invention, les moyens 64 permettant le verrouillage irréversible du corps secondaire 26 sur le corps principal 2 après injection de l'implant 6 pourront être combinés avec, voire remplacés par un système du type chemin de came 98. Ce chemin de came 98 est conçu de sorte que, tout en permettant le déplacement du corps principal 2 de sa position proximale à sa position distale et retour, il permette d'éviter le verrouillage involontaire du corps secondaire 26 sur le corps principal 2 avant injection tout en autorisant ce verrouillage après utilisation du dispositif d'injection 1 selon l'invention. Dans le cas où le système de verrouillage du type chemin de came 98 est utilisé en combinaison avec les moyens de verrouillage 64 décrits ci-avant, il permet de renforcer d'un point de vue mécanique l'immobilisation définitive du corps secondaire 26 sur le corps principal 2 après injection, rendant toute réutilisation du dispositif d'injection 1 ou simplement l'accès à l'aiguille 4 pratiquement impossible, sauf à détruire ledit dispositif d'injection 1.

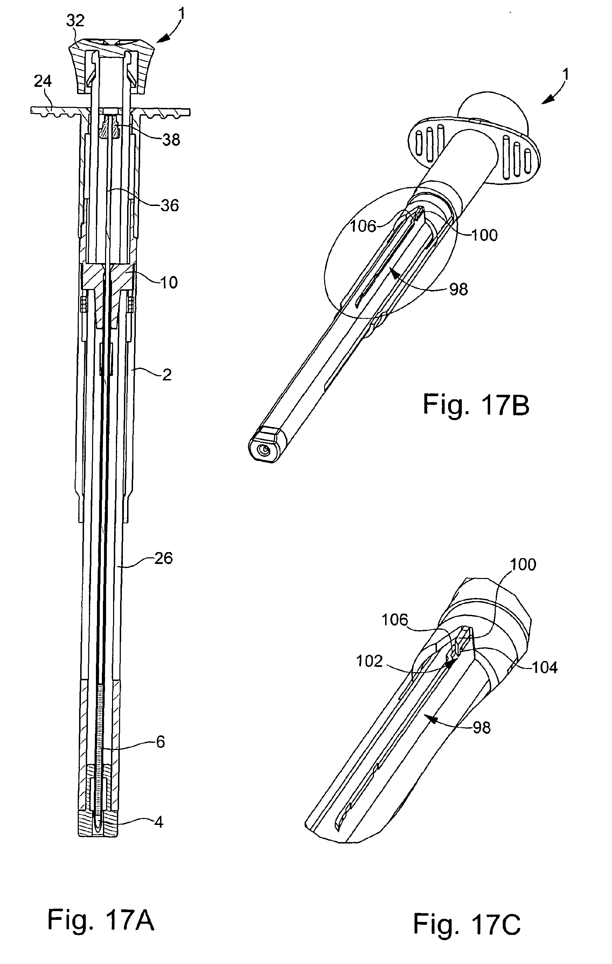

Dans l'exemple représenté au dessin (voir figures 17B et suivantes), le chemin de came 98 est ménagé sur la surface latérale extérieure du corps secondaire 26, s'étendant généralement rectilignement parallèlement à l'axe de symétrie longitudinale du corps secondaire 26. Le chemin de came 98 coopère avec des moyens flexibles, par exemple sous la forme d'une languette élastique 100, ménagée dans le corps principal 2 et qui est susceptible de se déformer transversalement de part et d'autre du chemin de came 98. Bien entendu, il pourrait également être prévu que le chemin de came 98 soit ménagé sur la face intérieure du corps principal 2 et que la languette 100 vienne de matière avec le corps secondaire 26.

Le chemin de came 98 que la languette élastique 100 ne pourra quitter lorsque le dispositif d'injection 1 est utilisé de façon normale, autrement dit sans forcer, est conçu de sorte que, lorsque ledit dispositif d'injection 1 est dans sa position de départ avant injection, la languette élastique 100 est en position de repos, calée dans un logement 102 (voir figures 17B et 17C). Ce logement 102 présente un retour 104 qui s'étend sensiblement perpendiculairement à la direction générale du chemin de came 98 et qui empêche le recul du corps principal 2 en direction du corps secondaire 26. De la sorte, le verrouillage involontaire du corps secondaire 26 sur le corps principal 1 par le biais des moyens de verrouillage 64 susdécrits lorsque ceux-ci sont prévus est impossible. Le logement 102 présente également une rampe 106 qui amène la languette 100 sur le chemin de came 98.

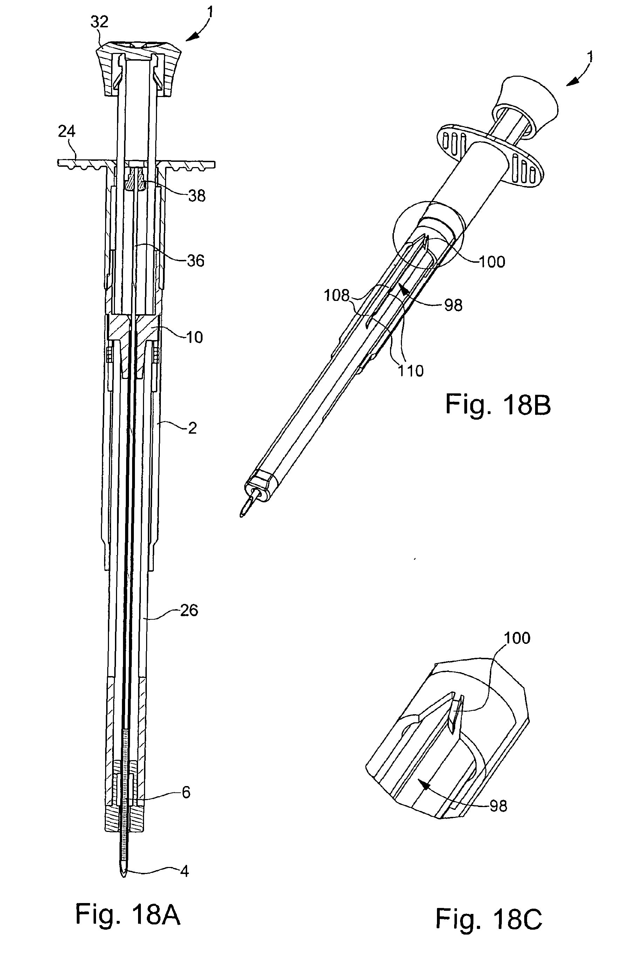

Quand on commence à faire coulisser le corps principal 2 le long du corps secondaire 26, la languette élastique 100 sort du logement 102 par le biais de la rampe 106 et commence à longer le chemin de came 98 (voir figures 18B et 18C). Du fait de la rampe 106 qu'elle a été obligée de suivre pour sortir du logement 102, la languette 100 est écartée de sa position de repos et se trouve contrainte élastiquement. Au fur et à mesure de sa progression le long du chemin de came 98, la languette élastique 100 franchit un ou plusieurs crans 108 prévus en différents endroits de la longueur dudit chemin de came 98 pour informer le praticien du bon déroulement du processus d'injection et de la pénétration de l'aiguille 4 dans la peau 50 du patient. De même, ces crans 108 empêchent d'effectuer le geste de rétro-injection avant que l'aiguille 4 ne soit complètement enfoncée dans les tissus 50. A chaque fois que la languette 100 tombe dans un cran 108, elle en ressort par le biais d'une rampe 110.

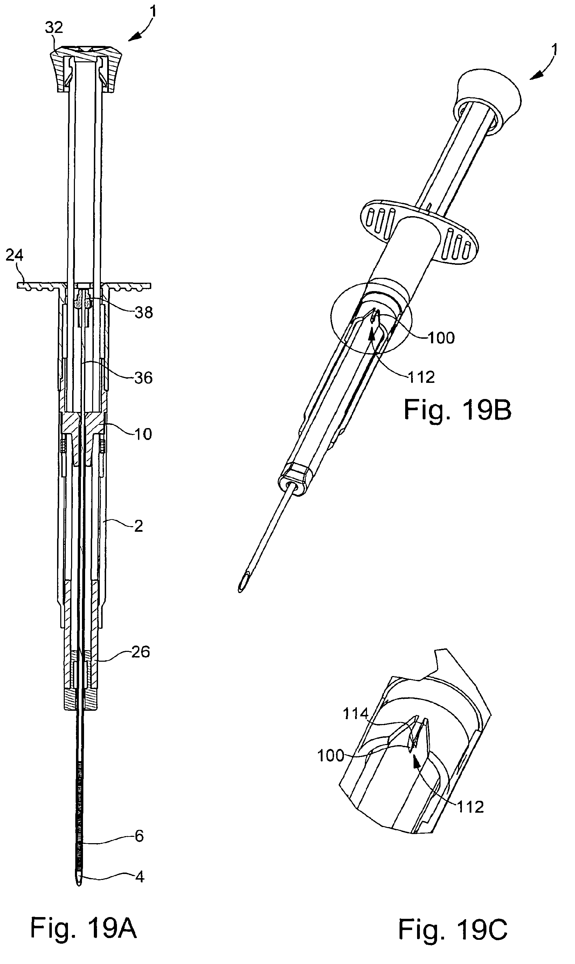

Lorsque le corps principal 2 arrive à sa position distale extrême et que l'aiguille 4 a complètement pénétré dans la peau 50 du patient, la languette 100 arrive au bout du chemin de came 98 au niveau d'un point 112 dit de rebroussement où elle se détend brusquement et où elle passe de l'autre côté dudit chemin de came 98 (voir figures 19B et 19C). Afin de rendre irréversible le passage de la languette élastique 100 de l'autre côté du chemin de came 98, ce dernier présente, au niveau de son point de rebroussement 112, une rampe 114 qui amène ladite languette 100 dans une position contrainte de sorte que, lorsque la languette 100 atteint ledit point de rebroussement 112, celle-ci revient temporairement dans une position de repos pour ensuite être à nouveau contrainte de l'autre côté du chemin de came 98 une fois le point de rebroussement 112 franchi. Le franchissement par la languette 100 du point de rebroussement 112 coïncide avec le moment où la tige de piston 36 se désolidarise du corps principal 2.

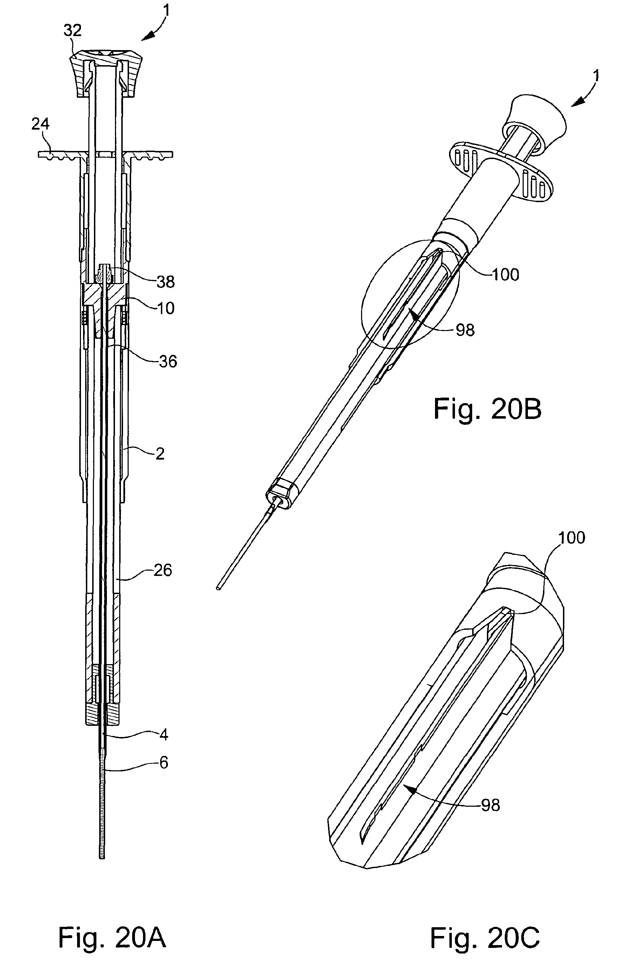

Lors du retour du corps principal 2 de sa position distale vers sa position proximale qui coïncide avec le retrait progressif de l'aiguille 4 hors de la peau 50 du patient, la languette élastique 100 parcourt, en sens contraire, un chemin parallèle à celui qu'elle avait emprunté lors de la phase de pénétration de l'aiguille 4 dans les tissus 50 (voir figures 20B et 20C).

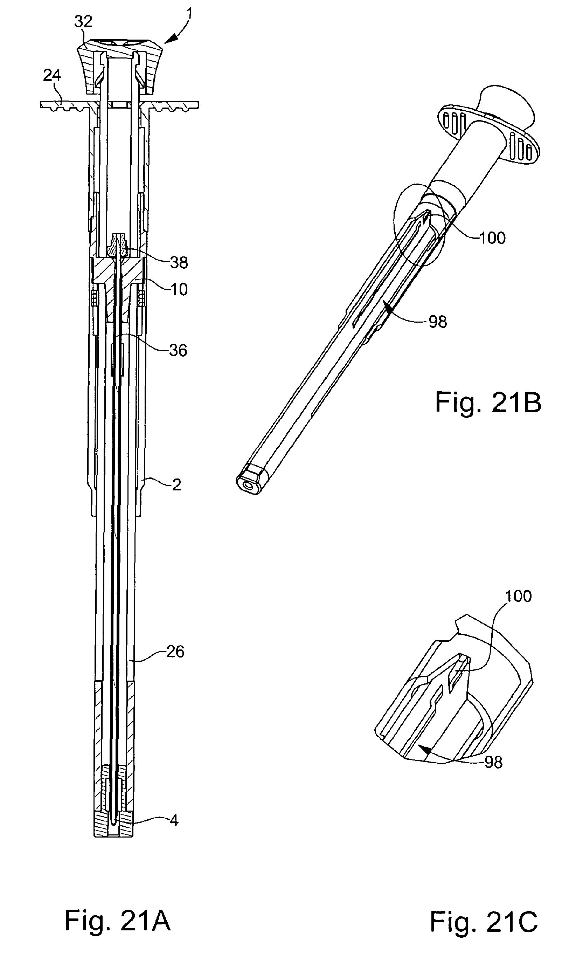

Le parcours de la languette 100 se poursuit jusqu'au moment où le corps principal 2 a regagné sa position proximale de départ dans laquelle le corps secondaire 26 peut se verrouiller sur ledit corps principal 2 si les moyens de verrouillage 64 ont été prévus à cet effet. Au même moment, la languette élastique 100 gagne un logement 116 dans lequel elle se bloque en position de repos (voir figures 21 B et 21 C).

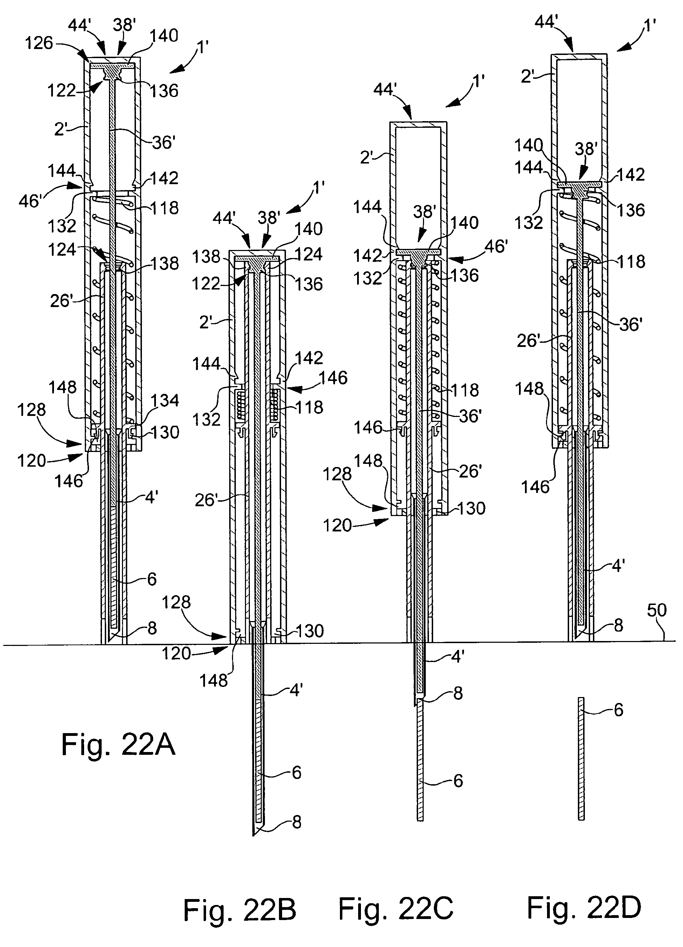

Avantageusement, le dispositif d'injection 1 selon l'invention peut, moyennant certaines adaptations, être automatisé par l'adjonction d'un ressort hélicoïdal 118 comme illustré aux figures 22A à 22D.

Conformément à ce mode de réalisation, le dispositif d'injection 1' comprend un corps principal creux 2' auquel est fixée de manière permanente, au niveau de sa partie distale 120, une aiguille creuse 4' dans laquelle est introduit un implant 6 destiné à être injecté.

Le corps principal creux 2' est apte à coulisser le long d'un corps secondaire 26', également creux, disposé coaxialement à l'intérieur dudit corps principal 2' et entourant l'aiguille 4' au moins jusqu'au biseau 8 de cette dernière.

Le dispositif d'injection 1' selon l'invention comprend enfin une tige de piston 36' susceptible de coulisser à l'intérieur de l'aiguille creuse 4' et qui sert à maintenir l'implant 6 à la bonne profondeur dans les tissus 50. Cette tige de piston 36' comprend une tête 38' équipée d'un moyen de blocage 122 destiné à coopérer avec un moyen de blocage correspondant 124 prévu à l'extrémité proximale du corps secondaire 26'.

La tige de piston 36' ne peut s'ébattre qu'entre une position proximale et une position distale contrôlées respectivement par une butée supérieure 44' et une butée inférieure 46' ménagées sur le corps principal 2'. Dans l'exemple représenté au dessin, la butée supérieure 44' est formée par l'extrémité proximale fermée du corps principal 2' contre laquelle la tige de piston 36' vient buter par sa tête 38'. Quant à la butée inférieure 46', elle est ménagée en un endroit de la longueur dudit corps principal 2', sur la surface latérale intérieure de ce dernier.

De même, le corps secondaire 26' ne peut s'ébattre qu'entre une position proximale et une position distale contrôlées respectivement par une butée supérieure 126 et une butée inférieure 128. Dans l'exemple représenté au dessin, la butée supérieure 126 est formée par l'extrémité proximale fermée du corps secondaire 2', et la butée inférieure 128 est constituée par des moyens de verrouillage irréversible 130 du corps secondaire 26' sur le corps principal 2'.

Le fonctionnement du dispositif d'injection 1' selon l'invention est le suivant. A la figure 22A, le dispositif d'injection 1' est représenté tel qu'il est livré au praticien, le corps secondaire 26' entourant l'aiguille 4' jusqu'à son biseau 8, de sorte que tout risque d'éraflure est évité. De même, l'aiguille 4' reste invisible au regard du patient. En tenant d'une main le dispositif d'injection 1' par son corps principal 2', le praticien vient appuyer l'extrémité distale du corps secondaire 26' contre la peau 50 du patient. Lorsque le dispositif d'injection 1 est convenablement disposé, le praticien exerce une poussée sur le corps principal 2'. Sous l'effet de cette poussée, le corps principal 2' se met à coulisser axialement le long du corps secondaire 26', permettant à l'aiguille 4' qui est solidaire dudit corps principal 2', de pénétrer dans la peau 50. Dans le même temps, le corps principal 2' entraîne la tige de piston 36' qui est en appui contre l'extrémité proximale fermée dudit corps principal 2', de sorte que la position relative de ladite tige 36' par rapport à l'aiguille 4' et à l'implant 6 reste inchangée. De même, le ressort 118 disposé coaxialement autour du corps secondaire 26' est maintenu axialement entre un épaulement annulaire 132 prévu sur la surface latérale intérieure du corps principal 1' et une collerette 134 prévue à l'extrémité distale du corps secondaire 26' se comprime.

Lorsque l'aiguille 4' est complètement enfoncée dans les tissus 50 (voir figure 22B), le corps secondaire 26' est venu se bloquer par ses moyens 124 sur les moyens de blocage correspondants 122 de la tige de piston 36'. Comme on peut le voir à l'examen des dessins, la tête 38' de la tige de piston 36' présente un corps de forme générale cylindrique comportant près de sa base un évidement circulaire formant un bourrelet 136 qui vient s'encliqueter en se déformant élastiquement dans une ouverture correspondante 138 ménagée à l'extrémité proximale du corps secondaire 26'. Dans cette position, le ressort 118 est comprimé.

A la figure 22C, le praticien relâche progressivement la pression qu'il exerce sur le corps principal 2'. Le ressort 118 se détend, entraînant avec lui le corps secondaire 26' qui lui-même entraîne la tige de piston 36'. Ainsi, la tige de piston 36' s'enfonce progressivement dans l'aiguille creuse 4' pour maintenir l'implant 6 à la bonne profondeur dans les tissus 50. Ce mouvement se poursuit jusqu'à ce que la tige de piston 36' vienne se bloquer contre la butée inférieure 46'. Comme on peut le voir à l'examen des dessins, la tête 38' de la tige de piston 36' comprend à son sommet une plaque circulaire 140 de diamètre sensiblement égal au diamètre intérieur du corps principal 1' et qui vient se loger dans une rainure 142 délimitée par un bourrelet 144 et l'épaulement annulaire 132.

A la figure 22D, l'opération d'injection est terminée. Le ressort 118 est à nouveau détendu, ayant amené le corps secondaire 26' en prise avec les moyens de verrouillage irréversible 130. Plus précisément, le corps secondaire 26' comprend à son extrémité distale au moins un et, préférentiellement, deux cliquets 146 qui se déforment élastiquement et viennent en prise sous un rebord 148 prévu sur la paroi latérale interne du corps principal 2', vers l'extrémité distale de ce dernier. Sauf à forcer et à détruire le dispositif d'injection 1', celui-ci est définitivement verrouillé et ne peut être réutilisé. L'aiguille 4' est à nouveau entourée par le corps secondaire 26', de sorte que tout risque de contamination par éraflure est évité. Avant utilisation, l'encliquetage involontaire des cliquets 146 sous le rebord 148 pourra être évité par adjonction d'un mécanisme du type chemin de came analogue à celui décrit précédemment.

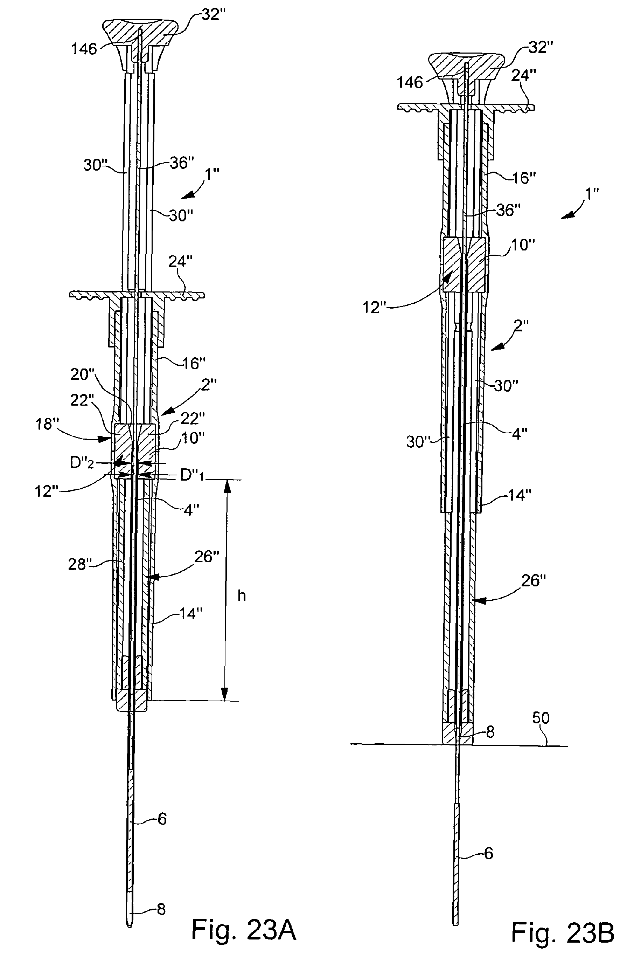

On représente en liaison avec les figures 23A et 23B une variante d'exécution simplifiée du dispositif d'injection 1 selon l'invention.

Désigné dans son ensemble par la référence numérique générale 1", le dispositif d'injection comprend un corps principal creux 2" auquel est fixée de manière permanente une aiguille creuse 4" dans laquelle est introduit un implant 6 destiné à être injecté. De préférence, l'aiguille creuse 4" présente à son extrémité distale un biseau 8 dont la géométrie est adaptée à l'application prévue pour le dispositif d'injection 1" et, en particulier, au mode d'administration. A son extrémité proximale, l'aiguille creuse 4" est montée fixe dans une pièce de maintien 10". Selon une première variante de réalisation, la pièce de maintien 10" est faite d'une pièce avec le corps principal creux 2". Selon une seconde variante de réalisation représentée au dessin, la pièce de maintien 10" sert de pièce de jonction à des parties tubulaires inférieure 14" et supérieure 16" qui constituent le corps principal creux 2" et dans lesquelles ladite pièce de maintien 10" est enchâssée. Une ouverture traversante 12" est pratiquée dans la pièce de maintien 10" pour le montage de l'aiguille creuse 4". Cette ouverture traversante 12" présente une première partie de diamètre D"₁ égal ou légèrement supérieur au diamètre extérieur de l'aiguille 4" pour permettre d'engager l'extrémité proximale de cette dernière dans la pièce de maintien 10". La première partie de diamètre D"₁ de l'ouverture traversante 12" est suivie d'une seconde partie de diamètre D"₂ inférieur au diamètre extérieur de l'aiguille 4" pour permettre le maintien de cette dernière dans la pièce de maintien 10" par frottement. Enfin, la seconde partie de diamètre D"₂ de l'ouverture traversante 12" est suivie d'une troisième partie en forme de cône qui va en s'évasant dans la direction de l'extrémité proximale du dispositif d'injection 1" selon l'invention.

La pièce de maintien 10" comprend une paroi latérale extérieure 18" qui délimite un volume intérieur dans lequel l'ouverture traversante 12" s'étend coaxialement. L'ouverture traversante 12" est matérialisée par une portion de tube 20" qui est reliée à la paroi extérieure 18" par au moins une et, préférentiellement, deux nervures radiales 22" diamétralement opposées.

De façon avantageuse, l'extrémité proximale du corps principal 2" est munie d'une pièce appuie-doigts 24" venant de matière avec ledit corps principal 2" ou se présentant sous forme d'une pièce rapportée.

Le corps principal creux 2" est apte à coulisser le long d'un corps secondaire 26", également creux, disposé coaxialement à l'intérieur dudit corps principal 2" et entourant partiellement l'aiguille 4". Ce corps secondaire 26" se présente sous la forme générale d'un tube 28" pourvu de deux fentes rectilignes 30" diamétralement opposées et qui s'étendent depuis l'extrémité proximale du tube 28" jusqu'à une hauteur h au-dessus de l'extrémité distale dudit tube 28". L'extrémité proximale du tube 28" sera avantageusement équipée de moyens facilitant la préhension du dispositif d'injection 1" tels qu'un bouton 32". Ce bouton 32" pourra être fait d'une pièce avec le corps secondaire 26" ou bien être réalisé sous forme d'une pièce rapportée.

Le dispositif d'injection 1" selon l'invention comprend enfin une tige de piston 36" susceptible de coulisser à l'intérieur de l'aiguille creuse 4" et qui sert à maintenir l'implant 6 à la bonne profondeur dans les tissus 50. Cette tige de piston 36" est rendue solidaire du corps secondaire 26" en étant chassée ou collée par son extrémité proximale dans un trou borgne 146 pratiqué dans le bouton 32".

Le principe de fonctionnement du dispositif d'injection 1" décrit ci-dessus est le suivant. A la figure 23A, le dispositif d'injection 1" est représenté tel qu'il est livré au praticien, avec l'aiguille 4" en position sortie. Le corps principal 2" occupe sa position distale extrême contrôlée par les nervures radiales 22" qui sont en butée contre le fond de fentes 30". Tenant d'une main le dispositif d'injection 1" par son corps principal 2", le praticien va enfoncer l'aiguille 4" dans les tissus 50. Une fois l'aiguille 4" complètement enfoncée dans les tissus 50, le praticien va procéder à une opération de rétro-injection. Plaçant son pouce sur le bouton 32", le praticien va commander la remontée du corps principal 2" en exerçant une traction sur l'appuie-doigts 24" qu'il tient de son index et de son majeur. La remontée du corps principal 2" s'accompagne du retrait progressif de l'aiguille 4" hors des tissus 50. Simultanément, la tige de piston 36" s'enfonce dans l'aiguille 4" pour maintenir l'implant 6 à la bonne profondeur dans les tissus 50. L'opération d'injection s'achève lorsque le corps principal 2" arrive à sa position proximale en butée contre le bouton 32" (voir figure 23B).

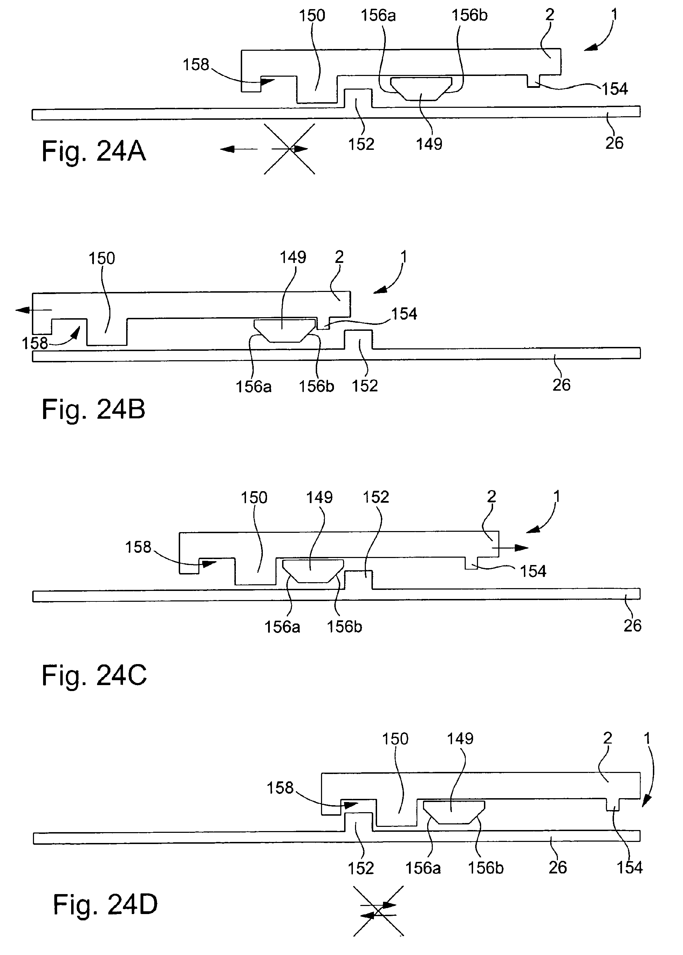

Les figures 24A à 24D illustrent schématiquement un autre mécanisme de verrouillage irréversible du corps secondaire 26 sur le corps principal 2. Ce mécanisme comprend essentiellement une bague 149 disposée librement entre le corps principal 2 et le corps secondaire 26.

A la figure 24A, le dispositif d'injection 1 est représenté tel qu'il est livré au praticien. Dans cette position, le corps principal 2 ne peut remonter en direction du corps secondaire 26, ce qui permet d'éviter tout verrouillage involontaire dudit corps principal 2 sur ledit corps secondaire 26. En effet, le corps principal 2 présente sur sa surface latérale intérieure un créneau 150 qui vient en butée contre un créneau correspondant 152 prévu sur la surface latérale extérieure du corps secondaire 26.

Lorsque l'on commence à faire pénétrer l'aiguille (non représentée) dans la peau, le corps principal 2 coulisse le long du corps secondaire 26, entraînant avec lui la bague 149 par le biais d'un créneau supplémentaire 154 prévu sur sa surface latérale intérieure (figure 24B). Lorsque la bague 149 arrive en butée contre le créneau 152, ce dernier se déforme élastiquement et passe sous ladite bague 149. Ce mouvement est facilité par la présence d'un plan incliné ou rampe 156a prévu sur la bague 149 et le long duquel le créneau 152 peut glisser.

Lorsque l'aiguille est complètement enfoncée dans la peau et que le corps principal 1 a atteint sa position distale extrême, on peut commencer l'opération de rétro-injection (figure 24C). Le corps principal 2 remonte le long du corps secondaire 26, entraînant avec lui la bague 149 par le biais de son créneau 150 en sens opposé au précédent jusqu'à ce que ladite bague 148 arrive en butée contre le créneau 152 du corps secondaire 26. A ce moment, le créneau 152 se déforme élastiquement, passant successivement sous la bague 148 et sous le créneau 150 et recouvre sa forme initiale en venant se loger dans une cavité 158 ménagée sur la surface latérale intérieure du corps principal 2 (figure 24D). Ce mouvement est facilité par la présence d'une deuxième rampe 156b prévue sur la bague 149 et le long de laquelle le créneau 152 peut glisser. A ce stade, le corps principal 2 et le corps secondaire 26 sont verrouillés en translation de manière irréversible et le dispositif d'injection 1 ne peut plus être réutilisé.

Il va de soi que la présente invention n'est pas limitée aux modes de réalisation qui viennent d'être décrits, et que diverses modifications et variantes simples peuvent être envisagées par l'homme du métier sans sortir du cadre des revendications annexées à la présente demande de brevet.

## Revendications

1. Dispositif d'injection d'un implant (6) dans des tissus (50), **caractérisé en ce qu'**il comprend un corps principal creux (2; 2') auquel est fixée une aiguille creuse (4; 4') dans laquelle est introduit l'implant (6), un corps secondaire (26; 26') disposé coaxialement à l'intérieur du corps principal (2;2') et entourant l'aiguille (4;4'), et une tige de piston (36; 36') susceptible de coulisser coaxialement à l'intérieur de ladite aiguille creuse (4; 4'), le dispositif d'injection (1; 1') étant agencé de façon que, lorsqu'il est pressé contre les tissus (50), ledit corps principal (2; 2') coulisse le long du corps secondaire (26; 26') depuis une position proximale vers une position distale pour permettre à l'aiguille (4; 4') de pénétrer dans lesdits tissus (50), le déplacement du corps principal (1; 1') s'accompagnant d'un déplacement concomitant de la tige de piston (36; 36'), et de façon que ladite tige de piston (36; 36') demeure fixe et maintienne l'implant (6) à une profondeur requise dans les tissus (50) jusqu'au retrait de l'aiguille (4; 4') hors desdits tissus (50) lors du retour du corps principal (2; 2') de sa position distale à sa position proximale.

2. Dispositif d'injection selon la revendication 1, **caractérisé en ce que** la position relative de la tige de piston (36; 36') et de l'implant (6) reste inchangée tout au long de l'opération d'injection.

3. Dispositif d'injection selon l'une quelconque des revendications 1 ou 2, **caractérisé en ce que** la position relative de la tige de piston (36; 36') et de l'aiguille creuse (4; 4') reste inchangée durant le déplacement du corps principal (2; 2') de sa position proximale vers sa position distale, et **en ce que** la tige de piston (36; 36') s'enfonce dans l'aiguille (4; 4') lors du retour du corps principal (2; 2') de sa position distale à sa position proximale.

4. Dispositif d'injection selon l'une quelconque des revendications 1 à 3, **caractérisé en ce que** la tige de piston (36; 36') ne peut s'ébattre qu'entre une position proximale et une position distale contrôlées respectivement par une butée supérieure (44; 44') et une butée inférieure (46; 46') ménagées sur le corps principal (2; 2').

5. Dispositif d'injection selon la revendication 4, **caractérisé en ce que** la tige de piston (36; 36') comprend une tête (38; 38') par laquelle elle coopère avec les butées supérieure (44; 44') et inférieure (46; 46').

6. Dispositif d'injection selon la revendication 5, **caractérisé en ce que** la tige de piston (36) est entraînée par le corps principal (2) lorsque celui-ci se déplace de sa position proximale vers sa position distale, et **en ce que** ladite tige de piston (36) est découplée dudit corps principal (2) lorsque ce dernier revient de sa position distale à sa position proximale.

7. Dispositif d'injection selon la revendication 6, **caractérisé en ce que**, lors du retour du corps principal (2) de sa position distale à sa position proximale, la tige de piston (36) reste fixe relativement audit corps principal (2), ladite tige (36) étant retenue par des moyens de blocage (34) prévus sur le corps secondaire (26).

8. Dispositif d'injection selon l'une quelconque des revendications 5 à 7, **caractérisé en ce que** les moyens de blocage (34) peuvent dépasser la tige de piston (36) lorsque cette dernière est en butée contre la butée supérieure (44) ou la butée inférieure (46).

9. Dispositif d'injection selon la revendication 8, **caractérisé en ce que** les moyens de blocage (34) sont aptes à se déformer élastiquement lorsqu'ils dépassent la tige de piston (36).

10. Dispositif d'injection selon l'une quelconque des revendications 7 à 9, **caractérisé en ce que** la tige de piston (36) coopère par sa tête (38) avec les moyens de blocage (34) du corps secondaire (26).

11. Dispositif d'injection selon la revendication 10, **caractérisé en ce que** les moyens de blocage (34) se présentent sous la forme d'un bourrelet prévu sur la face latérale interne du corps secondaire (26).

12. Dispositif d'injection selon l'une quelconque des revendications 5 à 11, **caractérisé en ce que** le corps principal (2) ne peut s'ébattre qu'entre une position proximale et une position distale qui sont contrôlées respectivement par une butée supérieure (40) et une butée inférieure (42) ménagées sur le corps secondaire (26).

13. Dispositif d'injection selon la revendication 12, **caractérisé en ce que** l'aiguille (4) est fixée sur le corps principal (2) par le biais d'une pièce de maintien (10).

14. Dispositif d'injection selon la revendication 13, **caractérisé en ce que** la pièce de maintien (10) comprend une portion de tube (20) qui délimite une ouverture traversante (12) pour la fixation de l'aiguille (4) et qui est reliée au corps principal (2) par au moins une nervure (22).

15. Dispositif d'injection selon la revendication 14, **caractérisé en ce que** la butée supérieure (40) est formée par un bouton (32) prévu à l'extrémité proximale du corps secondaire (26), et **en ce que** la butée inférieure (42) est définie par les nervures (22) qui viennent en butée contre le fond de fentes rectilignes longitudinales (30) prévues sur le corps secondaire (26) et qui s'étendent depuis l'extrémité proximale dudit corps secondaire (26) jusqu'à une hauteur (h) au-dessus de l'extrémité distale de ce dernier.

16. Dispositif d'injection selon l'une quelconque des revendications 13 à 15, **caractérisé en ce que** la butée supérieure (44) est constituée par des moyens (48) qui permettent le blocage du corps secondaire (26) sur le corps principal (2) avant injection, et **en ce que** la butée inférieure (46) est constituée par la pièce de maintien (10).

17. Dispositif d'injection selon la revendication 16, **caractérisé en ce que** les moyens de blocage (48) comprennent un anneau circulaire (56) relié au corps principal (2) par au moins un bras radial (58) et contre lequel la tête (38) de la tige de piston (36) vient buter, l'extrémité proximale (62) de ladite tête (38) faisant saillie dans l'anneau (56).

18. Dispositif d'injection selon la revendication 17, **caractérisé en ce qu'**il comprend en outre des moyens de verrouillage irréversible (64) du corps secondaire (26) sur le corps principal (2) après injection.

19. Dispositif d'injection selon la revendication 18, **caractérisé en ce que** les moyens de verrouillage (64) comprennent une paire de clips (66) ménagés sur le corps secondaire (26) et entre lesquels l'extrémité proximale (62) de la tête (38) de la tige de piston (36) est engagée pour empêcher ces clips (66) de pénétrer dans l'anneau circulaire (56) avant utilisation du dispositif d'injection (1), lesdits clips (66) n'étant plus entravés par ladite extrémité proximale (62) de la tête (38) de la tige de piston (36) après utilisation du dispositif d'injection (1) et pouvant ainsi faire saillie dans l'ouverture définie par l'anneau circulaire (56) pour verrouiller le corps secondaire (26) sur le corps principal (2).

20. Dispositif d'injection selon l'une quelconque des revendications 5 à 17, **caractérisé en ce qu'**il peut être chargé du côté de l'extrémité proximale du corps principal (2).

21. Dispositif d'injection selon la revendication 20, **caractérisé en ce qu'**au moins deux clips (74) ménagés sur le corps secondaire (26) coopèrent avec des éléments de retenue (76) ménagés à l'extrémité proximale du corps principal (2) pour permettre le verrouillage irréversible du corps secondaire (26) sur le corps principal (2) après utilisation du dispositif d'injection (1).

22. Dispositif d'injection selon l'une quelconque des revendications 20 ou 21, **caractérisé en ce que** le corps secondaire (26) comprend un bouton (32) présentant une ouverture traversante (88) par laquelle peuvent être successivement engagés l'implant (6) et la tige de piston (36).

23. Dispositif d'injection selon l'une quelconque des revendications 20 à 22, **caractérisé en ce que** la tête (38) de la tige de piston (36) et l'extrémité proximale du corps principal (2) sont agencées de façon à empêcher l'extraction de ladite tige de piston (36) une fois celle-ci introduite dans le corps principal (2).

24. Dispositif d'injection selon l'une quelconque des revendications 5 à 23, **caractérisé en ce qu'**il comprend un système à chemin de came (98) pour le verrouillage irréversible du corps secondaire (26) sur le corps principal (2).

25. Dispositif d'injection selon la revendication 24, **caractérisé en ce qu'**il comprend une languette élastique (100) qui coopère avec le chemin de came (98).

26. Dispositif d'injection selon la revendication 25, **caractérisé en ce qu'**avant utilisation, la languette (26) est calée dans un logement (102) de façon à empêcher le verrouillage involontaire du corps secondaire (26) sur le corps principal (2).

27. Dispositif d'injection selon l'une quelconque des revendications 25 ou 26, **caractérisé en ce que**, lorsqu'on commence à faire pénétrer l'aiguille (4) dans les tissus (50), la languette élastique (100) longe un côté du chemin de came (98) en étant contrainte élastiquement, arrive à un point de rebroussement (112) où elle se détend et où elle passe de l'autre côté dudit chemin de came (98) au moment où l'aiguille (4) a complètement pénétré dans les tissus (50), puis longe à nouveau le chemin de came (98) en sens contraire au précédent en étant contrainte élastiquement lorsqu'on retire l'aiguille (4) des tissus (50) jusqu'au moment où elle gagne un logement (116) dans lequel elle se bloque en position de repos, réalisant ainsi le verrouillage irréversible du corps secondaire (26) sur le corps principal (2).

28. Dispositif d'injection selon l'une quelconque des revendications 5 à 26, **caractérisé en ce qu'**il comprend un mécanisme de verrouillage irréversible à bague (148).

29. Dispositif d'injection selon la revendication 28, **caractérisé en ce que** la bague (148) est disposée librement entre le corps principal (2) et le corps secondaire (26) et coopère avec des créneaux (150, 154) et (152) prévus respectivement sur la surface latérale intérieure du corps principal (2) et sur la surface latérale extérieure du corps secondaire (26).

30. Dispositif d'injection selon la revendication 29, **caractérisé en ce que** lorsqu'on commence à faire pénétrer l'aiguille (4) dans les tissus (50), le corps principal (2) entraîne la bague (148) par le biais du créneau (154) jusqu'à ce que ladite bague (148) arrive en butée contre le créneau (152), ce dernier se déformant alors élastiquement et passant sous la bague (148), et **en ce que** lorsqu'on retire l'aiguille (4) des tissus (50), le corps principal (2) entraîne la bague (148) par le biais de son créneau (150) en sens opposé jusqu'à ce que ladite bague (148) arrive en butée contre le créneau (152) du corps secondaire (26), ce créneau (152) se déformant alors élastiquement et passant successivement sous la bague (148) et sous le créneau (150) et recouvrant sa forme initiale en venant se loger dans une cavité (158) ménagée sur la surface latérale intérieure du corps principal (2).

31. Dispositif d'injection selon la revendication 5, **caractérisé en ce qu'**il comprend un ressort (118) disposé coaxialement autour du corps secondaire (26') et maintenu axialement entre le corps principal (2') et le corps secondaire (26').

32. Dispositif d'injection selon la revendication 31, **caractérisé en ce que** le ressort (118) est maintenu axialement entre un épaulement annulaire (132) prévu sur la surface latérale intérieure du corps principal (2') et une collerette (134) prévue à l'extrémité distale du corps secondaire (26').

33. Dispositif d'injection selon l'une quelconque des revendications 31 ou 32, **caractérisé en ce que**, lorsque le corps principal (2') se déplace de sa position proximale vers sa position distale, le ressort (118) se comprime et la tête (38') de la tige de piston (36') vient se bloquer sur le corps secondaire (26'), de sorte que lorsque le corps principal (2') regagne sa position proximale, le ressort (118) se détend, entraînant avec lui le corps secondaire (26') qui lui-même entraîne la tige de piston (36').

34. Dispositif d'injection selon la revendication 33, **caractérisé en ce que** la tête (38') de la tige de piston (36') est pourvue d'un moyen de blocage (122) destiné à coopérer avec un moyen de blocage correspondant (124) prévu à l'extrémité proximale du corps secondaire (26').

35. Dispositif d'injection selon la revendication 34, **caractérisé en ce que** le moyen de blocage (122) est formé par un bourrelet (136) qui vient s'encliqueter dans une ouverture correspondante (138) ménagée à l'extrémité proximale du corps secondaire (26').

36. Dispositif d'injection selon l'une quelconque des revendications 31 à 35, **caractérisé en ce que** la butée supérieure (44') est formée par l'extrémité proximale du corps principal (2'), et **en ce que** la butée inférieure (46') est ménagée en un endroit de la longueur dudit corps principal (2'), sur la surface latérale intérieure de ce dernier.

37. Dispositif d'injection selon la revendication 36, **caractérisé en ce que** la butée inférieure (46') est formée par une rainure (142) dans laquelle la tête (38') de la tige de piston (36') vient se loger.

38. Dispositif d'injection selon l'une quelconque des revendications 31 à 37, **caractérisé en ce que** le corps secondaire (26') ne peut s'ébattre qu'entre une position proximale et une position distale contrôlées respectivement par une butée supérieure (126) et une butée inférieure (128).

39. Dispositif d'injection selon la revendication 38, **caractérisé en ce que** la butée supérieure (126) est formée par l'extrémité proximale du corps principal (2'), et **en ce que** la butée inférieure (128) est constituée par des moyens de verrouillage irréversible (130) du corps secondaire (26') sur le corps principal (2').

40. Dispositif d'injection selon la revendication 39, **caractérisé en ce que** les moyens de verrouillage (130) comprennent au moins un cliquet (146) prévu à l'extrémité distale du corps secondaire (26') et qui vient en prise sous un rebord (148) prévu sur la paroi latérale interne du corps principal (2').

41. Dispositif d'injection selon l'une quelconque des revendications 5 à 40, **caractérisé en ce que** le corps principal (2) est opaque et comprend une fenêtre à travers laquelle la tête (38) de la tige de piston (36) est visible après utilisation, ce qui indiquera que le dispositif d'injection (1) est déchargé.

42. Dispositif d'injection selon l'une quelconque des revendications 5 à 40, **caractérisé en ce que** la pièce appuie-doigts (24) est opaque et comprend une fenêtre à travers laquelle la tête (38) de la tige de piston (36) apparaît avant injection, indiquant que le dispositif d'injection (1) est chargé, et **en ce que** la tête (38) de la tige de piston (36) apparaît dans une seconde fenêtre pratiquée dans le corps principal opaque (2) pour indiquer que le dispositif d'injection (1) a été utilisé et est donc vide.

43. Dispositif d'injection selon l'une quelconque des revendications 5 à 40, **caractérisé en ce qu'**une fenêtre s'étend depuis la position qu'occupe la tête (38) de la tige de piston (36) lorsque le dispositif d'injection (1) est chargé jusqu'à la position qu'occupe cette tête (38) lorsque ledit dispositif d'injection (1) est vide.

44. Dispositif d'injection d'un implant (6) dans des tissus (50), **caractérisé en ce qu'**il comprend un corps principal creux (2") auquel est fixée une aiguille creuse (4") dans laquelle est introduit l'implant (6) et qui fait saillie du corps principal (2"), un corps secondaire (26") disposé coaxialement à l'intérieur du corps principal (2"), et une tige de piston (36") susceptible de coulisser coaxialement à l'intérieur ladite aiguille creuse (4"), le dispositif d'injection (1") étant agencé de façon que, lorsqu'on exerce une pression sur le corps principal (2") pour faire pénétrer l'aiguille (4") dans les tissus (50), la tige de piston (36") demeure fixe et maintient l'implant (6) à une profondeur requise dans les tissus (50) jusqu'au moment où l'aiguille (4") est retirée des tissus (50), ce retrait étant commandé par ledit corps principal (1 ") qui coulisse le long du corps secondaire (26") depuis une position initiale dans laquelle ledit corps principal (2") bute contre une extrémité distale du corps secondaire (26"), jusqu'à une position finale dans laquelle ledit corps principal (2") bute contre une extrémité proximale du corps secondaire (26"), la tige de piston (36") pénétrant alors progressivement dans l'aiguille creuse (4").

45. Dispositif d'injection selon la revendication 44, **caractérisé en ce qu'**après injection, le corps secondaire (26") entoure l'aiguille (4").

46. Dispositif d'injection selon l'une quelconque des revendications 44 ou 45, **caractérisé en ce que** la tige de piston (36") est solidaire du corps secondaire (26").

47. Dispositif d'injection selon l'une quelconque des revendications 44 à 46, **caractérisé en ce que** l'aiguille (4") est fixée sur le corps principal (2") par le biais d'une pièce de maintien (10").

48. Dispositif d'injection selon la revendication 47, **caractérisé en ce que** la pièce de maintien (10") comprend une portion de tube (20") qui délimite une ouverture traversante (12") pour la fixation de l'aiguille (4") et qui est reliée au corps principal (2") par au moins une nervure (22").

49. Dispositif d'injection selon la revendication 48, **caractérisé en ce que** la position initiale du corps principal (2") est définie par les nervures qui viennent en butée contre le fond de fentes rectilignes longitudinales (30") prévues sur le corps secondaire (26") et qui s'étendent depuis l'extrémité proximale dudit corps secondaire (26") jusqu'à une hauteur (h) au-dessus de l'extrémité distale de ce dernier.

50. Dispositif d'injection selon l'une quelconque des revendications 44 à 49, **caractérisé en ce que** le corps principal (2") comprend une pièce appuie-doigts (24") et **en ce que** le corps secondaire (26") comprend un bouton (32").
